# EUROPEAN PATENT APPLICATION

(11) **EP 4 289 330 A1**
(43) Date of publication of application: **13.12.2023**
(21) Application number: 22177865.7
(22) Date of filing: 08.06.2022
(51) Int. Cl.: A61B 1/00, A61B 1/06, A61B 1/05

(54) **VIDEO LARYNGOSCOPE**

(71) Applicant: Ambu A/S, 2750 Ballerup (DK)
(72) Inventor: PILBEAM, Christopher, Jordan, 2720 Vanløse (DK); SELVÖ, Berth Thomas Fredrik, 24235 Hörby (SE); ORTEGA ZAFRA, Sebastian, 21758 Malmö (SE); JIN, Jing, 23737 Bjärred (SE)
(74) Representative: COPA Copenhagen Patents

(57) **Abstract**

Disclosed is a laryngoscope assembly (4) and parts thereof. The laryngoscope assembly comprises a blade unit (100) extending from a proximal end (102) to a distal end (104), the blade unit having a distal part (106) extending to the distal end and comprising a first surface (108), wherein the distal part is adapted to be inserted into a patient's oral cavity with the first surface facing the patient's tongue. The laryngoscope assembly further comprises a control unit (200) comprising a body portion (202) having a first body portion (202A) and a second body portion (202B), the first body portion being couplable to the blade unit. The control unit and the blade unit, when being coupled, collectively form a laryngoscope. The control unit being adapted to receive image data from an image sensor (402) as the image data is being generated by the image sensor, wherein, at least when the control unit is coupled to the blade unit, the image data is indicative of a view from the distal part of the blade unit in a direction at least partly towards the distal end.

## Description

The present disclosure relates to a visualization device, such as a video laryngoscope and a system comprising such visualization device. The present disclosure also relates to accompanying devices and elements associated with such device and system.

### BACKGROUND

A medical visualization device may be utilized to visually examine certain areas of the body of a person, such as inside a body cavity of the person. For example, a visualization device may be used to inspect the airways, the digestive tract, or the intestines. For example, a visualization may be formed of one or more disposable parts and optionally one or more reusable parts.

A visualization device may be provided with a camera and be attached to a monitor device, such as a monitor with a display screen. Alternatively, or additionally, the visualization device may itself be provided with a display unit with a display screen. A video output from the camera of the visualization device may be received and displayed at the monitor device and/or at the display unit, thereby allowing an operator to control the visualization device to inspect an area of interest. For example, an operator may use the visualization device, particularly when the visualization device is a laryngoscope, to perform an intubation procedure of a patient.

### SUMMARY

The present disclosure relates to a visualization device, such as a laryngoscope. A laryngoscope with a camera may conventionally be denoted a video laryngoscope. However, in the present disclosure the pre-fix "video" in "video laryngoscope" may be omitted while still referring to a laryngoscope with a camera.

It is an objective of the present application to provide improvements or at least alternatives to presently known techniques within the field of video laryngoscopes.

The present disclosure comprises a visualization device, such as a video laryngoscope and a system, such as a laryngoscope system, comprising such visualization device. The present disclosure also relates to accompanying devices and elements associated with such device and system, such as a monitor device, a battery charger etc.

The present invention is further defined by the appended set of claims.

### GENERAL DESCRIPTION

A control unit for a laryngoscope is disclosed. A blade unit for a laryngoscope is disclosed. A laryngoscope assembly comprising the control unit and the blade unit is disclosed. The blade unit may be disposable, i.e. intended for single use. The control unit may be reusable, i.e. intended for being used several times, e.g. with different blade units.

The blade unit is extending from a proximal end to a distal end. The blade unit has a distal part extending to the distal end and comprising a first surface. The distal part is adapted to be inserted into a patient's oral cavity with the first surface facing the patient's tongue.

The control unit comprises a body portion having a first body portion and a second body portion. The first body portion is couplable to the blade unit.

The control unit and the blade unit, when being coupled, collectively form the laryngoscope. The laryngoscope has a handle portion extending along a first axis. In some examples, the blade unit may comprise the handle portion. In other examples, the control unit may comprise the handle portion. A forward direction of the laryngoscope is perpendicular to the first axis in a direction towards the distal end of the blade unit. A rearward direction of the laryngoscope is opposite the forward direction. The forward direction and/or the rearward direction may be extending radially from the first axis. The forward direction and the rearward direction are extending in a laryngoscope mid-plane defined by the first axis and the distal end of the blade unit.

The control unit is adapted to receive image data from an image sensor as the image data is being generated by the image sensor. At least when the control unit is coupled to the blade unit, the image data is indicative of a view from the distal part of the blade unit in a direction at least partly towards the distal end.

The control unit may comprise electronic circuitry, such as a control unit processing unit and/or memory, such as volatile and/or non-volatile memory. The control unit processing unit may be adapted to receive the image data from the image sensor.

The control unit may comprise a control unit display element. The control unit display element may comprise a control unit display. The control unit, such as the control unit processing unit, may be adapted to cause the control unit display to display a live representation of the image data.

The control unit may comprise a connector cable. The connector cable may be adapted to connect the control unit to a monitor device. The monitor device may be an external component comprising a monitor display, which may be caused to display the live representation of the image data. The monitor device may comprise electronic circuitry, such as a monitor processing unit and/or monitor memory, such as volatile and/or non-volatile memory.

The control unit may comprise a battery, e.g. a rechargeable battery, being adapted to power the control unit. The battery may further be adapted to power the image sensor. The battery may further be adapted to power an optional light emitter. The battery may be part of a battery pack, which will be described later.

The control unit may comprise the image sensor. For example, the image sensor may be arranged at a distal end of the first body portion, e.g. at the end of an extension portion of the control unit. The extension portion may be a flexible portion. Thereby, the image sensor may be arranged in the distal part of the blade unit when the control unit is coupled to the blade unit.

Alternatively, the blade unit may comprise the image sensor. The image sensor may be located in the distal part of the blade unit. The control unit may comprise an electrical control unit connector at the distal end of the first body portion. The blade unit may comprise an electrical blade connector. The electrical control unit connector may be adapted to electrically connect the control unit to the image sensor of the blade unit, e.g. by connecting the electrical control unit connector and the electrical blade connector.

The second body portion may have an attachment part being positioned in the rearward direction relative to the first axis.

The control unit display element may be attached, such as hingedly attached, to the second body portion, such as to the attachment part of the second body portion. Alternatively or additionally, the connector cable may be attached to the attachment part of the second body portion. The connector cable may extend from the second body portion in a direction substantially parallel to the first axis.

The second body portion may comprise a control button with a button surface being positioned in the forward direction relative to the attachment part.

With the disclosed arrangement of the control button, the control button is provided in a more ergonomic position. The whole device will therefore sit more sturdily in the user's hand. Furthermore, by the disclosed arrangement of the control button, the user may preserve a firm grip of the device, by the thumb and three other fingers, while operating the control button with the index finger. Hence the disclosed arrangement provides for a confident grip of the device while operating the control button.

The disclosed solution is also advantageous in that the control button is positioned in the forward direction relative to the control unit display, in the case of the control unit comprising the control unit display element, thereby, operating the control button will not obstruct the view of the control unit display.

The button surface may have a button surface normal, e.g. at the center of the control button. An angle between the button surface normal and the forward direction may be less than 90 degrees, such as less than 80 degrees. An angle between the button surface normal and the first axis may be between 0-180 degrees, such as between 1-179 degrees. The angle between the button surface normal and the first axis may be less than 90 degrees.

The button surface may comprise a tactile protrusion. The tactile protrusion may be located substantially at the center of the control button. Provision of a tactile protrusion gives the user tactile feedback for locating the control button with the index finger, without having to look for it. Users of laryngoscopes often wear gloves, which reduces sensitivity of the fingers, so distinct tactile feedback provided by the tactile protrusion is advantageous.

The control unit processing unit may be adapted to receive an electronic signal from the control button indicative of the control button being depressed.

The control unit, such as the control unit processing unit, may be adapted to detect a first user input corresponding to a first press of the control button (e.g. a short press, such as a press of the control button having a duration shorter than a press threshold, e.g. 1 s). In accordance with detection of the first user input, the control unit, such as the control unit processing unit, may cause recording of a first image file corresponding to the image data received when the first user input was detected.

The control unit, such as the control unit processing unit, may be adapted to detect a second user input corresponding to a second press of the control button (e.g. a long press, such as a press of the control button having a duration longer than the press threshold). In accordance with detection of the second user input, the control unit, such as the control unit processing unit, may cause initiating a recording of a first video file corresponding to the image data received after detection of the second user input. After detection of the second user input, the control unit, such as the control unit processing unit, may be adapted to detect a third user input corresponding to the second press of the control button. In accordance with detection of the third user input, the control unit, such as the control unit processing unit, may cause ending the recording of the first video file. Thereby the first video file may correspond to the image data received between detection of the second user input and the third user input.

The first image file and/or the first video file may be recorded in the memory of the control unit.

In some examples, the control unit, such as the control unit processing unit, may be adapted to forward the electronic signal from the control button to the monitor device, such as the monitor processing unit of the monitor device, in particular in examples wherein the control unit comprises a connector cable connecting the control unit to the monitor device. The control button of the control unit may work as a remote control for one or more functions of the monitor device. The monitor device, such as the monitor processing unit, may receive the electronic signal from the control button (or another signal indicative of the electronic signal from the control button) indicative of the control button being depressed.

The monitor device, such as the monitor processing unit, may be adapted to detect a first user input corresponding to a first press of the control button (e.g. a short press, such as a press of the control button having a duration shorter than a press threshold, e.g. 1 s). In accordance with detection of the first user input, the monitor device, such as the monitor processing unit, may cause recording of a first image file corresponding to the image data received when the first user input was detected.

The monitor device, such as the monitor processing unit, may be adapted to detect a second user input corresponding to a second press of the control button (e.g. a long press, such as a press of the control button having a duration longer than the press threshold). In accordance with detection of the second user input, the monitor device, such as the monitor processing unit, may cause initiating a recording of a first video file corresponding to the image data received after detection of the second user input. After detection of the second user input, the monitor device, such as the monitor processing unit, may be adapted to detect a third user input corresponding to the second press of the control button. In accordance with detection of the third user input, the monitor device, such as the monitor processing unit, may cause ending the recording of the first video file. Thereby the first video file may correspond to the image data received between detection of the second user input and the third user input.

The first image file and/or the first video file may be recorded in the monitor memory of the monitor device.

The control button may have a control button light indicator. The control button light indicator may be adapted to signal various information to the user. For example, the control unit, such as the control unit processing unit, may be adapted to detect coupling with the blade unit. In accordance with detection of coupling between the blade unit and the control unit, the control unit, such as the control unit processing unit, may provide a first visual indication with the control button light indicator (e.g. steadily lit). In accordance with detection of no coupling between the blade unit and the control unit, the control unit, such as the control unit processing unit, may provide a second visual indication (e.g. blinking or breathing) with the control button light indicator.

Hence, the present disclosure provides a dual functionality of the control button and allows the control button to convey additional information to the user. Thereby the user may be conveniently informed of the functioning of the device.

The control unit may comprise a power button (e.g. an on/off button). The control unit may comprise a battery light indicator. The control unit, such as the control unit processing unit, may be adapted to detect a first user input corresponding to a first press of the power button. In accordance with detection of the first user input, the control unit, such as the control unit processing unit, may provide a battery status indication with the battery light indicator.

The control unit, such as the control unit processing unit, may be further adapted to detect a second user input corresponding to a second press of the power button. In accordance with detection of the second user input, the control unit, such as the control unit processing unit, may turn on/off the control unit.

The first press of the power button may correspond to a short press of the of the power button. For example, the first press of the power button may be a press of the power button having a duration shorter than a press threshold, e.g. 1 s.

The second press of the power button may correspond to a long press of the of the power button. For example, the second press of the power button may be a press of the power button having a duration longer than the press threshold

By the present disclosure, the user may easily check the present battery capacity by shortly pressing the power button. Hence, the user may be provided information of the battery capacity, and thereby whether needing to charge or replace the battery, without having to power on the control unit. Thereby, checking the battery capacity is less time consuming and consumes less power from the battery.

The power button and/or the battery light indicator may be arranged on the control unit display element. For example, on a side of the control unit display element.

The power button may comprise the battery light indicator. Alternatively, the battery light indicator may be located near the power button, such as next to the power button, on a same side of the control unit, such as on a same side of the control unit display element.

The battery status indication may have a duration between 0.5-2.0 seconds.

The control unit, such as the control unit processing unit, may be further adapted to determine a remaining battery capacity of the battery, e.g. by interrogating an integrated battery gauge. In accordance with the remaining battery capacity being less than a low battery threshold (e.g. 20% of full charge) the battery status indication may comprise cyclically lighting the battery light indicator a plurality of times (e.g. blinking for the duration of the battery status indication) with a first color (e.g. orange). Preferably the first color is not red, as the color red often is associated with an emergency, e.g. a life-threatening situation. In accordance with the remaining battery capacity being more than the low battery threshold and less than a high battery threshold (e.g. 50%) the battery status indication may comprise lighting the battery light indicator once (e.g. being constantly lit for the duration of the battery status indication) with the first color. In accordance with the remaining battery capacity being more than the high battery threshold the battery status indication may comprise lighting the battery light indicator once (e.g. being constantly lit for the duration of the battery status indication) with a second color. The second color may be different from the first color. For example, the second color may be green.

Also, a camera unit for a laryngoscope is disclosed. The camera unit may form part of the blade unit or may form part of the control unit. The camera unit comprises a camera unit housing. The camera unit housing comprises a first side wall and a second side wall. The first side wall and the second side wall may be parallel. The camera unit housing comprises a third side wall extending between the first side wall and the second side wall. The third side wall may be perpendicular to the first side wall and/or the second side wall. The camera unit housing comprises a fourth side wall extending between the first side wall and the second side wall. The fourth side wall may be perpendicular to the first side wall and/or the second side wall. The fourth side wall may be parallel to the third side wall. The camera unit housing further comprises a separating wall extending between the first side wall and the second side wall and being arranged between the third side wall and the fourth side wall. The separating wall may be perpendicular to the first side wall and/or the second side wall. The separating wall may be parallel to the third side wall and/or the fourth side wall. The separating wall may be light-tight.

The camera unit comprises an image sensor being adapted to generate image data indicative of a view along a viewing direction from a front surface of the image sensor. The image sensor may be accommodated in a first housing compartment of the camera unit housing defined by the first side wall, the second side wall, the third side wall and the separating wall.

The camera unit may comprise a light emitter, e.g. an LED. The light emitter may be adapted to emit light along the viewing direction from a front surface of the light emitter. The light emitter may be accommodated in a second housing compartment of the camera unit housing defined by the first side wall, the second side wall, the fourth side wall and the separating wall.

By separating the light emitter and image sensor with the separating wall, light from the light emitter is prevented or restricted from entering the compartment of the image sensor, which would impact the image quality. Thus, by the present solution, the image quality obtained by the image sensor may be improved.

A length of the separating wall along the viewing direction may be more than 1.5 mm, such as more than 2 mm.

The camera unit may be arranged at the distal end of the first body portion, e.g. at the end of the extension portion of the control unit, such that when the control unit and the blade unit are coupled, the camera unit is arranged in the distal part of the blade unit with the viewing direction being in a direction at least partly towards the distal end of the blade unit. Thereby, the camera unit may provide the image sensor at the distal end of the first body portion, e.g. at the end of the extension portion of the control unit, as described previously. Alternatively, the camera unit may be arranged in the blade unit, such as in the distal part of the blade unit, with the viewing direction being at least partly towards the distal end of the blade unit. Thereby, the camera unit may provide the image sensor in the distal part of the blade unit, as described previously. Thus, the image sensor of the camera unit may form the image sensor of the laryngoscope when the camera unit forms part of the control unit and/or the blade unit. Thus, the viewing direction, when the camera unit is arranged as intended in the blade unit (e.g. when the control unit and blade unit is coupled) may be from the distal part of the blade unit in the direction towards the distal end of the blade unit.

The camera unit housing may comprise a front side wall, e.g. extending between the first side wall and the second side wall and between the third side wall and the fourth side wall. The front side wall may have a first window and a second window. The separating wall may adjoin the front side wall between the first window and the second window. The first window may provide an optical connection between the first housing compartment through the front side wall. The second window may provide an optical connection between the second housing compartment through the front side wall.

The image sensor may be arranged with a distance between the front surface of the image sensor and the first window. The distance between the front surface of the image sensor and the first window may be between 0.1 mm and 0.5 mm, such as between 0.2 mm and 0.4 mm.

The light emitter may be arranged with a distance between the front surface of the light emitter and the second window. The distance between the front surface of the light emitter and the second window may be between 0.1 mm and 0.5 mm, such as between 0.2 mm and 0.4 mm.

The inventors have found it advantageous to have the distance between the window and the light emitter slightly larger than the distance between the image sensor and the window. Thus, the distance between the front surface of the image sensor and the first window may be smaller than the distance between the front surface of the light emitter and the second window.

The image sensor and the light emitter may be arranged with an offset distance along the viewing direction between the front surface of the image sensor and the front surface of the light emitter. The light emitter may be arranged behind the image sensor along the viewing direction. The offset distance may be between 0.1 mm - 1.5 mm, such as between 0.5 mm and 1 mm.

When arranged in the blade unit, such as in the distal part of the blade unit, the camera unit may be arranged opposite the first surface of the distal part of the blade unit.

When arranged in the blade unit, such as in the distal part of the blade unit, the camera unit may be arranged with a blade tip distance between the front surface of the image sensor and the distal end of the blade unit. The blade tip distance may be between 19-50 mm. In some examples, the blade tip distance may be between 20-30 mm, such as between 24-26 mm. In some examples, the blade tip distance may be between 15-25 mm, such as between 18-22 mm. In some examples, the blade tip distance may be between 30-40 mm, such as between 34-36 mm. In some examples, the blade tip distance may be between 40-50 mm, such as between 44-46 mm or between 46-49 mm.

The control unit may comprise a battery receiver adapted to receive a replaceable battery pack. The replaceable battery pack may comprise the battery as previously mentioned. The battery pack may be a rechargeable battery pack, i.e. the battery may be a rechargeable battery. Hence, the battery pack may comprise a rechargeable battery.

The battery pack may be adapted to power the control unit. The battery pack may be adapted to power the image sensor and/or the camera unit. The battery receiver may comprise a battery connector with a plurality of battery connector terminals. The battery connector terminals may be adapted to cooperate with corresponding connection terminals of the replaceable battery pack.

The battery receiver may comprise an outer connector seal encircling the battery connector. The outer connector seal may be adapted to, when the replaceable battery pack is received in the battery receiver, contact a corresponding surface of the replaceable battery pack encircling the connection terminals of the replaceable battery pack.

By this disclosed solution, a water-tight seal encircling the terminals connecting the battery pack and the control unit may be provided, protecting the electrical connection between the battery pack and the control unit from being disrupted by water or other liquid.

The replaceable battery pack may be devoid of an outer sealing element adapted to contact a surface of the control unit. Thereby, the battery pack may be less expensive to manufacture, as a sealing element may be omitted.

The control unit may comprise an internal sealing encircling the battery connector. The internal sealing may prevent liquid from entering from the battery receiver and into an internal space of the control unit when the replaceable battery pack is not received in the battery receiver.

The control unit may comprise a battery connector circuitry. The battery connector circuitry may be arranged inside the control unit. The battery connector may be attached to a first side of the battery connector circuitry. The internal sealing may be arranged between the battery connector circuitry and a shell element of the control unit forming part of the battery receiver.

The internal sealing may prevent liquid from entering and potentially damaging internal components of the control unit, when the outer sealing element is not preventing liquid from entering the battery connector because of the battery pack not being received in the battery received.

The battery receiver may form part of the first body portion. The first body portion may be adapted to be received and enclosed by the handle portion of the blade unit. Thereby, the battery pack, when being received in the battery receiver, may be enclosed, such as fully enclosed, by the handle portion of the blade unit. Thereby, the battery pack may be protected, which may be particularly advantageous in case of a rechargeable battery pack, which may be intended to be used many times and potentially in combination with several control units.

The blade unit at the proximal end may comprise a blade coupling portion. The blade coupling portion may be adapted to couple the blade unit to a control unit, such as the control unit as disclosed herein, to form the laryngoscope. The blade coupling portion may comprise a channel extending along a channel axis to receive a first body portion of the control unit, such as the first body portion of the control unit as previously described.

The handle portion of the blade unit may form the channel. Hence, the channel axis may be the same as the first axis, as previously described.

The channel may be formed of a first channel part and a second channel part. The second channel part may be between the proximal end of the blade unit and the first channel part. The second channel part may comprise or form the proximal end of the blade unit. The first channel part and the second channel part may be connected along a connected portion between a first angular position and a second angular position about the channel axis. The first channel part and the second channel part may be separated, e.g. by a slit, along a slit portion extending from the second angular position to the first angular position about the channel axis. Hence, for example, together the slit portion and the connected portion may completely surround the channel axis. The slit portion may be longer than the connected portion. For example, the slit portion may extend along more than 70% of the circumference of the channel. In some examples, the slit portion may extend along between 50-80% of the circumference of the channel, such as between 65-75% of the circumference of the channel.

The second channel part, e.g. as an effect of the slit portion, may be more flexible than the first channel part, which may be substantially rigid.

The second channel part may comprise at least one retention element. The at least one retention element may be between a first retention element angular position and a second retention element angular position about the channel axis. The first retention element angular position and the second retention element angular position may be within the slit portion. An angular distance between the first retention element angular position and the first angular position may be at least 45 degrees, such as at least 70 degrees. An angular distance between the second retention element angular position and the second angular position may be at least 45 degrees, such as at least 70 degrees. The at least one retention element may comprise a protrusion or a recess. The at least one retention element may be facing the channel axis and/or may be provided on a surface of the second channel part facing the channel axis. The at least one retention element may be adapted to engage with one or more cooperating retention elements of the first body portion of the control unit.

The angular distance between the first retention element angular position and the first angular position may be substantially the same as the angular distance between the second retention element angular position and the second angular position.

The blade unit may, as previously mentioned, comprise an image sensor, and optionally a light emitter (e.g. as part of the described camera unit), being arranged in the distal part with a viewing direction being in a direction at least partly towards the distal end of the blade unit. The blade unit may comprise an electrical blade connector comprising one or more terminals electrically connected to the image sensor, and optionally the light emitter. The electrical blade connector may be adapted to electrically connect the image sensor, and optionally the light emitter, with the control unit couplable to the blade unit. The electrical blade connector may be arranged in the channel. The electrical blade connector may, preferably, be arranged at a distal end of the channel opposite the proximal end of the blade unit.

The first channel part may comprise one or more alignment features adapted to engage with one or more cooperating alignment features of the first body portion of the control unit. The one or more alignment features may, preferably, be one or more protrusions, e.g. elongated protrusions. Alternatively, the one or more alignment features may be one or more slits. The one or more alignment features may be facing the channel axis and/or may be provided on a surface of the first channel part facing the channel axis.

The first body portion of the control unit may extend along a first body portion axis. When the first body portion is received in the channel of the blade unit the first body portion axis may be substantially parallel to the channel axis. The first body portion may comprise one or more cooperating retention elements adapted to engage with the at least one retention element of the second channel part. The one or more cooperating retention elements may comprise a protrusion or a recess. Preferably, if the at least one retention element of the second channel part of the blade unit comprises a protrusion, the one or more cooperating retention elements may comprise a cooperating recess. Similarly, if the at least one retention element of the second channel part of the blade unit comprises a recess, the one or more cooperating retention elements may comprise a cooperating protrusion.

The first body portion may have a first width at a first position. The first position may be between a proximal end of the first body portion and an intermediate position. The proximal end of the first body portion may be adjoining the second body portion. The intermediate position may be along the first body portion axis. The first body portion may have a second width at a second position between the intermediate position and a distal position along the first body portion axis. The second width may be greater than the first width. The slimmer part of the first body portion, e.g. at the first position, may make space for deflection of the sides of the second channel part, when disengaging the blade unit and the control unit.

The first body portion may comprise one or more cooperating alignment features adapted to engage with the one or more alignment features of the first channel part. The one or more cooperating alignment features may be provided between the intermediate position and the distal position along the first body portion axis. The one or more cooperating alignment features may, preferably, be slits extending along the first body portion axis. Alternatively, the one or more cooperating alignment features may be recesses. Preferably, if the at least one alignment features of the first channel part are one or more protrusion, the one or more cooperating alignment features may be one or more cooperating recesses. Similarly, if the at least one alignment features of the first channel part are one or more recesses, the one or more cooperating alignment features may be one or more cooperating protrusions.

A laryngoscope system is also disclosed. The laryngoscope system may comprise the blade unit as previously described. The laryngoscope system may comprise the control unit as previously described. The laryngoscope system may comprise a monitor device, which may be couplable to the blade unit via the control unit, when the control unit having a connector cable for connecting the control unit to the monitor device.

The laryngoscope system may comprise the blade unit, as described, and an image sensor, e.g. as part of the control unit or as part of the blade unit. The laryngoscope may comprise a display, which may be the control unit display of the control unit, or the monitor display of the monitor device.

The laryngoscope system, such as a processing unit of the laryngoscope system, such as the control unit processing unit of the control unit or the monitor processing unit of the monitor device, may display a live representation of the image data with the display.

The laryngoscope system, such as a processing unit of the laryngoscope system, such as the control unit processing unit of the control unit or the monitor processing unit of the monitor device, may further detect a trigger-event, and in response to detection of the trigger event: start a timer function and display, concurrently with the live representation, a value of the timer function with the display.

Some guidelines specify that an operator should revert to alternative methods for ensuring ventilation of the patient, if intubation has not been completed within a predefined amount of time, e.g. within 30 seconds. Thus, the disclosed solution is advantageous in that the operator is provided information of the current situation and may be notified when having to revert to other options. Thus, observance of guidelines may be assisted and potentially be enhanced.

In some examples, the timer function is a countdown, e.g. decreasing towards zero. Such countdown may start from a pre-defined amount of time, e.g. according to a guideline's pre-defined amount of time before reverting to alternative methods. The pre-defined amount of time may, for example, be 30 seconds. In other examples, the timer function may be a timer starting from zero and counting upwards.

The laryngoscope system, such as a processing unit of the laryngoscope system, such as the control unit processing unit of the control unit or the monitor processing unit of the monitor device, may detect coupling between the control unit and the blade unit. The trigger event may correspond to coupling of the blade unit and the control unit.

Alternatively, or additionally, the trigger event may correspond to a user input, such as a press of a button or a tap on the display.

The laryngoscope system, such as a processing unit of the laryngoscope system, such as the control unit processing unit of the control unit or the monitor processing unit of the monitor device, may detect, e.g. by image analysis of the image data, insertion of the distal part of the blade unit into a patient's oral cavity. The trigger event may correspond to insertion of the distal part of the blade unit into a patient's oral cavity, e.g. as detected by the laryngoscope system.

After start of the timer function, in accordance with the value of the timer function being outside (e.g. more than) a low-threshold time range (e.g. 5 seconds), the laryngoscope system, such as a processing unit of the laryngoscope system, such as the control unit processing unit of the control unit or the monitor processing unit of the monitor device, may display the value of the timer function having a first visual characteristic. In accordance with the value of the timer function being within (e.g. less than) the low-threshold time range, the laryngoscope system, such as a processing unit of the laryngoscope system, such as the control unit processing unit of the control unit or the monitor processing unit of the monitor device, may display the value of the timer function having a second visual characteristic different from the first visual characteristic. For example, the second visual characteristic may be a different color, e.g. orange, and/or the second visual characteristic may be blinking. Such change of visual characteristic may emphasize to the user that the time limit is approaching.

A person skilled in the art will appreciate that any one or more of the above aspects of this disclosure and embodiments thereof may be combined with any one or more of the other aspects of this disclosure and embodiments thereof.

### BRIEF DESCRIPTION OF THE FIGURES

Embodiments of the disclosure will be described in more detail in the following with regard to the accompanying figures. The figures show one way of implementing the present invention and are not to be construed as being limiting to other possible embodiments falling within the scope of the attached claim set.
Figs. 1A and 1B schematically illustrates an exemplary laryngoscope system,
Fig. 2 schematically illustrates an exemplary laryngoscope system,
Figs. 3A-3D schematically illustrates different views of an exemplary blade unit,
Figs. 4A and 4B schematically illustrate an exemplary control unit,
Figs. 5A and 5B schematically illustrate an exemplary control unit,
Fig. 6 schematically illustrates an exemplary laryngoscope,
Fig. 7 schematically illustrates an exemplary laryngoscope,
Fig. 8 schematically illustrates an exemplary laryngoscope assembly,
Fig. 9 is a block diagram schematically illustrating an exemplary laryngoscope assembly,
Fig. 10 is a block diagram schematically illustrating an exemplary visualization system,
Fig. 11 schematically illustrates an exemplary camera unit,
Figs. 12A and 12B schematically illustrate cross-sectional views of exemplary camera units,
Fig. 13 schematically illustrates an exemplary control unit,
Fig. 14 schematically illustrates an exemplary battery pack,
Fig. 15 schematically illustrates a cross-sectional view of an exemplary battery connector.
Fig. 16 schematically illustrates an exemplary laryngoscope system,
Figs. 17A and 17B schematically illustrate an exemplary control unit,
Figs. 18A and 18B schematically illustrate an exemplary an exemplary electrical control unit connector,
Fig 19 schematically illustrates an exemplary carry case for an exemplary control unit, and
Fig. 20 schematically illustrates an exemplary battery charger for an exemplary control unit.

### DETAILED DESCRIPTION

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

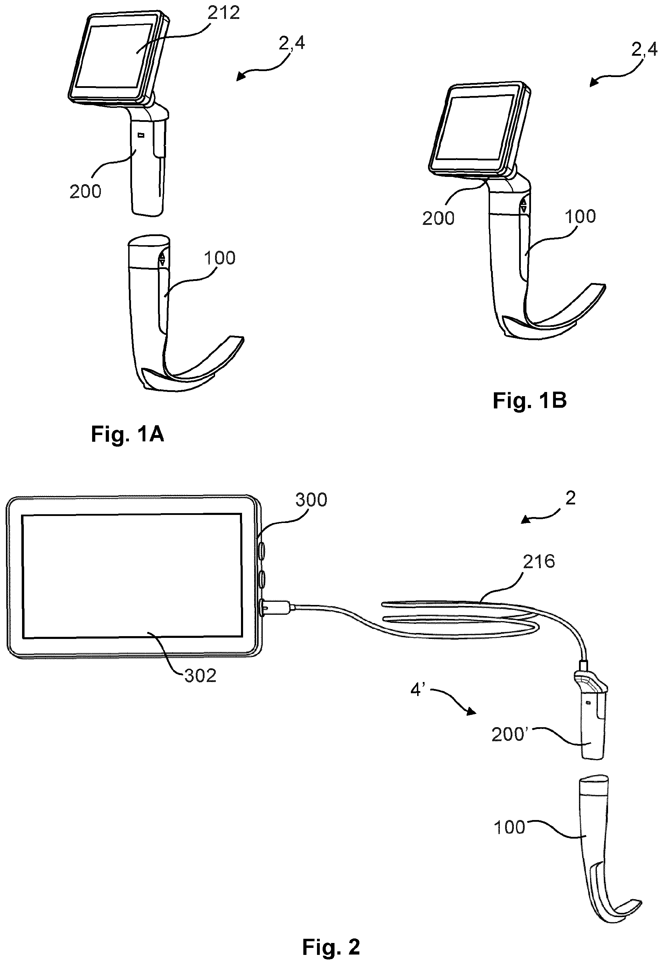

Figs. 1A and 1B schematically illustrate an exemplary laryngoscope system 2 comprising an exemplary laryngoscope assembly 4. The laryngoscope assembly 4 comprises a blade unit 100. The blade unit 100 is adapted to be at least partly inserted into a patient's oral cavity, during an intubation procedure. The blade unit 100 may have a variety of shapes, for example, such as those known in the art as MAC, Miller, and hyper-angulated. The blade unit 100 may also come in various sizes, conventionally denoted as size 00, size 0, size 1, size 2, size 3, size 4 and size 5. The laryngoscope assembly 4 further comprises a control unit 200 with a control unit display 212. The control unit display 212 may be a touch sensitive display. The control unit 200 is couplable to the blade unit 100, as illustrated in Fig. 1B. The control unit 200 and the blade unit 100, when being coupled, collectively form a laryngoscope. The control unit 200 is adapted to receive image data from an image sensor located near the tip of the blade unit 100, at least when the control unit 200 is coupled to the blade unit 100, and to cause the control unit display 212 to display a live representation of the image data. Thereby, an operator may be assisted by a video feed from the tip of the blade unit during the intubation procedure.

Fig. 2 schematically illustrates an exemplary laryngoscope system 2 comprising an exemplary laryngoscope assembly 4' and an exemplary monitor device 300. The elements depicted in Fig. 1 and the elements depicted in Fig. 2 may form part of the same laryngoscope system 2. Furthermore, the laryngoscope system 2 may comprise additional blade units, endoscopes, and/or other elements not depicted.

The laryngoscope assembly 4' comprises a blade unit 100, which may be the same or a similar blade unit 100 as described and illustrated in relation to Figs. 1A and 1B. The laryngoscope assembly 4' further comprises a control unit 200' connectable, e.g. with a connector cable 216 as illustrated, to the monitor device 300. The control unit 200' is couplable to the blade unit 100, similar to the control unit 200 of Figs. 1A and 1B. The control unit 200' and the blade unit 100, when being coupled, collectively form a laryngoscope. The control unit 200' is adapted to receive image data from the image sensor located near the tip of the blade unit 100 and to transmit the image data or other data indicative of the received image data to the monitor device 300. The monitor device 300 may display with the monitor display 302 (and/or with an external display not shown) the live representation of the image data.

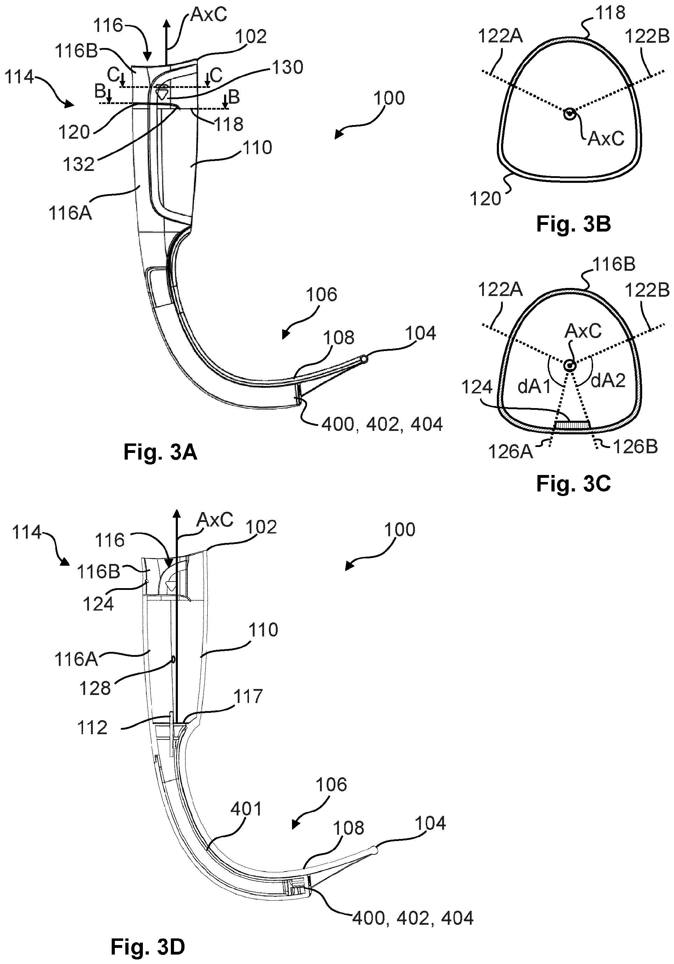

Fig. 3A schematically illustrates an exemplary blade unit 100, such as the blade unit as exemplified and described in relation to Figs. 1-2. The blade unit 100 extends from a proximal end 102 to a distal end 104. The blade unit has a distal part 106 extending to the distal end 104 and comprising a first surface 108. The distal part 106 is adapted to be inserted into a patient's oral cavity with the first surface 108 facing the patient's tongue. The first surface 108 may alternatively be denoted an inferior surface, as it will be facing inferiorly with respect to the patient's anatomy when in use.

The blade unit 100, in the illustrated example, comprises a handle portion 110 to form a handle portion of the laryngoscope. However, in other exemplary blade units the handle portion may be omitted from the blade unit 100 and a handle portion may instead be provided by the control unit 200 couplable to the blade unit 100. The handle portion 110 may comprise different textures to provide for an enhanced grip. Furthermore, the handle portion 110 may comprise sunken parts. For example, the sunken parts of the handle portion 110 may comprise a rougher texture than the remainder of the handle portion 110. The rough texture may enhance the grip. The smoother parts of the handle portion may facilitate production as it may, in case of, e.g. injection molding, facilitate removal of the halves from the mold.

The blade unit 100, e.g. one or more shell parts of the blade unit 100, may be injection molded. For example, the blade unit 100 may comprise two halves, which may be injection molded. The two halves may be joined by means of ultrasonic welding and/or by adhesive.

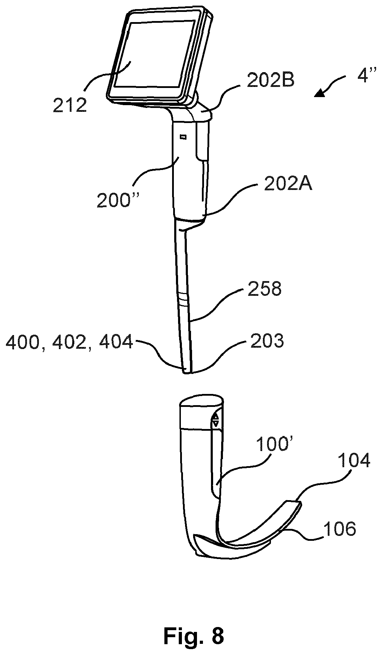

The blade unit 100 may further comprise a camera unit 400 comprising an image sensor 402 and optionally a light emitter 404. In some examples the camera unit 400 may form part of the blade unit 100, as illustrated, however, alternatively, the camera unit 400 may form part of the control unit, as further explained in relation to Fig. 8. The image sensor is adapted to generate image data indicative of a view from the distal part 106 of the blade unit 100.

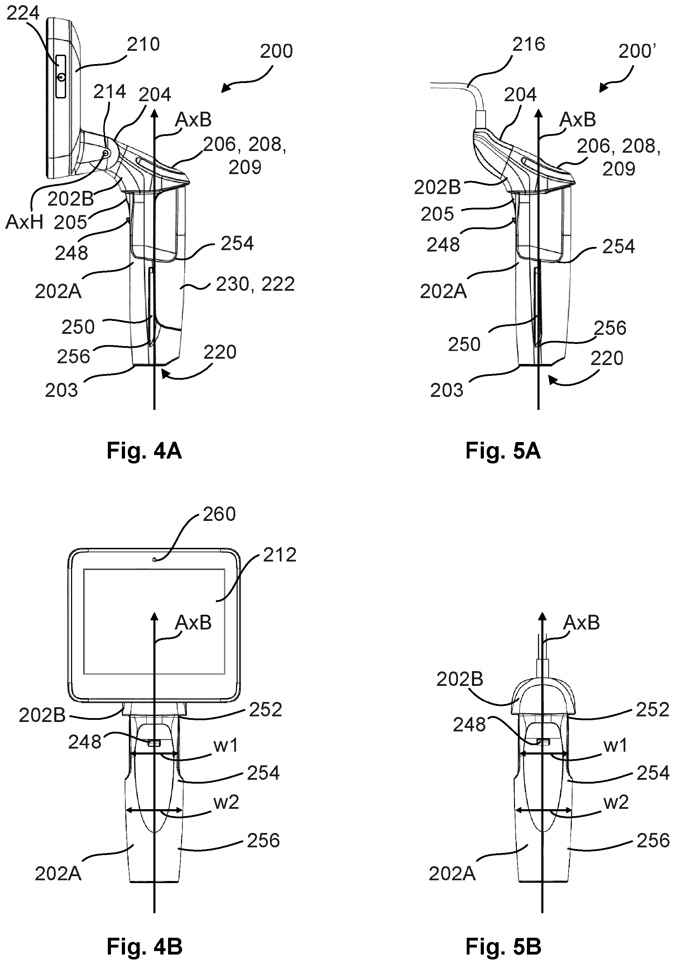

Figs. 4A and 4B schematically illustrate an exemplary control unit 200, such as the control unit 200 as exemplified and described in relation to Figs. 1A and 1B. Figs. 5A and 5B schematically illustrate an exemplary control unit 200', such as the control unit 200' as exemplified and described in relation to Fig. 2. In most aspects control unit 200 and control unit 200' comprise identical or very similar features. Thus, in the following Figs. 4A, 4B, 5A and 5B will be described together.

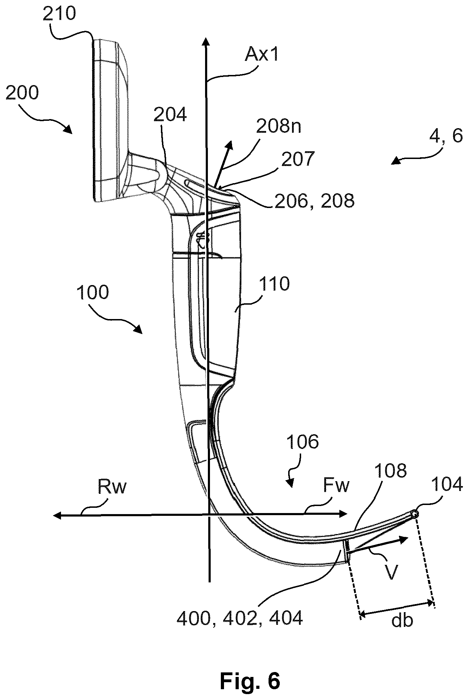

The control unit 200, 200' comprises a body portion 202 having a first body portion 202A and a second body portion 202B. The first body portion 202A is couplable to the blade unit 100, e.g. as illustrated in Fig. 1B. In some examples, the first body portion 202A may be adapted to be received and enclosed by a handle portion 110 of the blade unit 100 (see, e.g. Fig. 6). The control unit 200, 200' may comprise a ramp 205, which can support the second channel part 116B from the inside. This may lower stresses in and deformation of the blade if the blade is pulled without correctly disengaging.

The second body portion 202B has an attachment part 204. The attachment part 204 may provide for attachment to a display element 210, as is the case for control unit 200, or the attachment part 204 may provide for attachment to a connector cable 216 as is the case for control unit 200'.

The control unit 200 comprises a control unit display element 210 with a control unit display 212 (see Figs. 1A/1B). In this example, the control unit display element 210 is attached to the attachment part 204 of the second body portion 202B. The control unit display element 210 may be hingedly attached to the attachment part 204. Hence, the control unit display element 210 and the attachment part 204 may be connected by a hinge 214. The control unit display element 210 may be rotatable about a hinge axis AxH relative to the second body portion 202B, such as relative to the attachment part 204 of the second body portion 202B.

The control unit 200' comprises a connector cable 216 adapted to connect the control unit 200' to a monitor device 300 (see Fig. 2). In this example, the connector cable 216 is attached to the attachment part 204 of the second body portion 202B.

The second body portion 202B of the control unit 200, 200' comprises a control button 206 with a button surface 208. The control unit 200, 200' may be adapted to execute functions, such as storing still images and/or video, in response to receipt of various user inputs, e.g. short press or long press, of the control button 206. The control button may further comprise a control button light indicator 209. The control button light indicator 209 may be used to indicate various information associated with the control unit 200, 200', such as, for example, whether a blade unit 100 is coupled or not.

The control unit 200 comprises a battery pack 230. The battery pack may comprise a battery 222, such as a rechargeable battery. The battery pack 230 may be replaceable. The battery pack 230 being replaceable provides for a backup solution in case of low power, in that a spare battery pack may be kept with the control unit 200.

The battery 222 may be adapted to power the control unit 200. The battery 222 may further be adapted to power the image sensor 402 and/or the light emitter 404. For example, the battery 222 may be adapted to power electronics of the blade unit 100, such as the image sensor 402 and the light emitter 404 of the blade unit 100, when the control unit 200 and the blade unit is coupled. The control unit 200' may omit a battery and/or battery pack, as it may receive power from the monitor device. However, in some examples, the control unit 200' may similarly comprise a battery pack 230 and/or a battery 222.

The control unit 200, 200' comprises an electrical control unit connector 220 at a distal end 203 of the first body portion 202A. The electrical control unit connector 220 is adapted to electrically connect the control unit 200, 200' to the camera unit 400, image sensor 402 and/or light emitter 404 of the blade unit 100. Furthermore, the electrical control unit connector 220 may be used for connecting a data transfer cable to the control unit 200, 200', e.g. to allow transfer of data from internal memory of the control unit 200, 200' to an external device, such as a laptop, a smart phone, a tablet, etc.

The control unit 200 comprises a power button 224. The control unit 200 may comprise a battery light indicator, which may form part of the power button or be separate thereto. For example, the battery light indicator may be provided by the display of the control unit display element 210 or the power button 224 may comprise the battery light indicator. The power button 224 and/or the battery light indicator may be arranged on the control unit display element 210, e.g. on a side of the control unit display element 210, as illustrated. The power button 224 and the battery light indicator may be arranged on the same side of the control unit display element 210, e.g. next to each other. The power button 224 may be used to turn on/off the power of the control unit 200 and/or to check a current status of the battery 222. The control unit 200' may omit a power button 224, as it may receive power from the monitor device, and hence may be turned on whenever it is connected to the monitor device. However, in some examples, the control unit 200' may similarly comprise a power button 224. Particularly, if the control unit 200' comprises a battery pack 230 and/or a battery 222, it may be relevant to provide the control unit 200' also with the disclosed power button 224.

The control unit 200 may comprise a light sensor 260. The control unit 200 may be adapted to automatically adjust brightness of the control unit display 212 in accordance with the amount of ambient light. For example, if the control unit 200 is used in a setting with much ambient light, e.g. outside, the brightness of the control unit display 212 may be high, whereas in a setting with little ambient light, e.g. inside, such as in a dark room, the brightness of the control unit display 212 may be low. An icon or alert on the screen may be shown when the screen brightness is adjusted and to what level it has been adjusted to. Additionally, or alternatively, the user may have a possibility, e.g. via a touch interface on the control unit display 212 to manually adjust the brightness of the control unit display 212. For example, the user may turn up brightness by swiping upwards on the control unit display 212 and turn down brightness by swiping downwards on the control unit display 212.

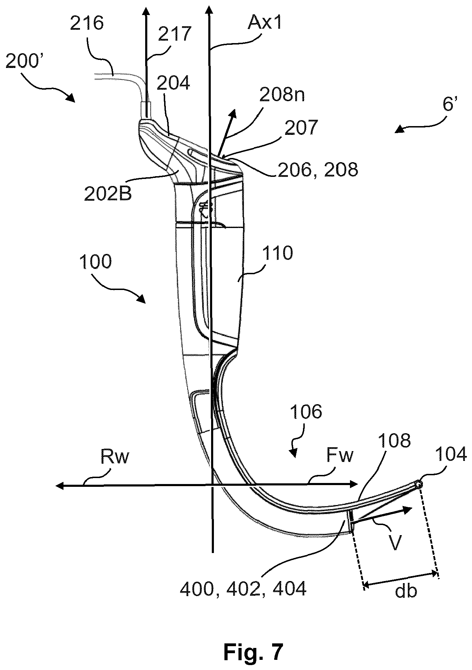

Referring again to Fig. 3A, at the proximal end 102, the blade unit 100 comprises a blade coupling portion 114 adapted to couple the blade unit 100 to the control unit 200, 200' to form the laryngoscope 6, 6' (see Figs. 6 and 7). The blade coupling portion 114 comprises a channel 116 extending along a channel axis AxC to receive the first body portion 202A of the control unit 200, 200' (see Figs. 4A, 4B, 5A and 5B). The first body portion 202A of the control unit 200, 200' may extend along a first body portion axis AxB, as illustrated in Figs. 4A, 4B, 5A and 5B. When the first body portion 202A is received in the channel 116 of the blade unit 100, the first body portion axis AxB may be substantially parallel to the channel axis AxC.

The channel 116 is formed of a first channel part 116A and a second channel part 116B. The second channel part 116B is between the proximal end 102 of the blade unit 100 and the first channel part 116A. Preferably the second channel part has a dimension to fit a human finger, such as a minimum distance between the proximal end 102 of the blade unit and the first channel part 116A of approx. 1 cm. The first channel part 116A and the second channel part 116B are connected along a connected portion 118 and separated along a slit portion 120.

Fig. 3B schematically illustrates a cross-sectional view of the exemplary coupling portion 114 along the line B-B in Fig. 3A. The connected portion extends from a first angular position 122A to a second angular position 122B about the channel axis AxC. The slit portion 120 extends from the second angular position 122B to the first angular position 122A about the channel axis AxC. The slit portion 120 may be longer than the connected portion 118, i.e. the angular distance between the first angular position 122A and the second angular position 122B along the connected portion 118 may be less than the angular distance between the first angular position 122A and the second angular position 122B along the slit portion 120. For example, the angular distance between the first angular position 122A and the second angular position 122B along the connected portion 118 may be less than the 180 degrees, such as less than 150 degrees. In some examples, the slit portion 120 may extend about more than 70% of the circumference of the channel 116, e.g. for increased flexibility of the second channel part 116B.

Fig. 3C schematically illustrates a cross-sectional view of the exemplary coupling portion 114 along the line C-C in Fig. 3A. The second channel part 116B comprises a retention element 124 between a first retention element angular position 126A and a second retention element angular position 126B about the channel axis AxC.

The retention element 124 may be a protrusion, as illustrated. Alternatively, the retention element may be a recess. The retention element 124 is provided on an inner surface of the second channel part 116B and facing the channel axis AxC. The first body portion 202A of the control unit 200, 200' may comprise one or more cooperating retention elements 248 (see Figs. 4A, 4B, 5A and 5B). The retention element 124 is adapted to engage with the one or more cooperating retention elements 248 of the first body portion 202A of the control unit 200, 200'.

The first retention element angular position 126A and the second retention element angular position 126B are within the slit portion 120. An angular distance dA1 between the first retention element angular position 126A and the first angular position 122A is at least 45 degrees, such as at least 70 degrees. An angular distance dA2 between the second retention element angular position 126B and the second angular position 122B is at least 45 degrees, such as at least 70 degrees. The angular distance dA1 between the first retention element angular position 126A and the first angular position 122A and the angular distance dA2 between the second retention element angular position 126B and the second angular position 122B may be substantially the same.

The slit portion 120 between the first channel part 116A and the second channel part 116B provides a flexibility of the second channel part 116B, allowing the wall of the second channel part 116B comprising the retention element 124 to move radially outwards, i.e. away from the channel axis AxC, thereby allowing the retention element 124 to pass over the cooperating retention element 248 of the control unit 200, 200'. Furthermore, by the present arrangement, the retention element 124 may be forced radially outwards, i.e. away from the channel axis, by the user pushing radially inwards on the sides of the second channel part 116B, e.g. at a pinch point indicator 130. Hence, the user may disengage the blade unit 100 from the control unit 200, 200' by pinching the second channel part 116B. The ends of the slit 120 are preferably curved downwards, e.g. comprising a curved end 132, away from the opening of the channel 116, so stress built-up in the material and potential cracks are directed away from the opening. Flexibility of the second channel part 116B is increased for increased length of the slit portion, so the pinch force to disengage the blade from the control unit can be tailored to the needs.

Referring to Figs 4B and 5B, the first body portion 202A of the control unit 200, 200' may be thinner along the part being located in the second channel part 116A, when being received in the channel 116, such as to allow the sides of the second channel part 116B to be deflected inwards, when disengaging the blade unit and the control unit 200, 200'. The first body portion 202A may have a first width w1 at a first position between a proximal end 252 of the first body portion 202A adjoining the second body portion 202B and an intermediate position 254 along the first body portion axis AxB. The first body portion may have a second width w2 greater than the first width w1. The second width w2 is at a second position between the intermediate position 254 and a distal position 256 along the first body portion axis AxB. The second width may decrease towards the distal position 256. The width of the first body portion 202A may decrease towards the distal portion 256 and/or towards the distal end 203. The width of the first body portion 202A may decrease to facilitate easier insertion of the first body portion 202A in the channel 116 of the blade unit 100.

As also visible in Figs. 3B and 3C, the blade unit 100, more specifically the channel 116, may have a cross-sectional shape, which is not rotational symmetrical. For example, the cross-sectional shape of the channel 116 may be a somewhat triangular shape, as illustrated. The first body portion 202A of the control unit 200, 200' (see Figs. 4A-5B) may have a corresponding cross-sectional shape, such that the first body portion 202A can be received in the channel 116. The cross-sectional shape may have one, such as only one, symmetry line.

The cross-sectional shape, by being not rotational symmetrical, facilitates easy and correct coupling of the control unit 200, 200' and the blade unit 100. An incorrect engagement of the two components will be noticed by the user at an early stage of assembling, as the incorrect coupling will be problematic and non-intuitive already from the beginning of engagement.

Furthermore, the exemplary shape of the channel 116 and the first body portion 202A may provide for a shape of the handle portion 110, which conforms to the palm of a hand.

Fig. 3D schematically illustrates a cross-sectional view of the blade unit 100 of Fig. 3A. The first channel part 116A may comprise one or more alignment features 128. The first body portion 202A of the control unit 200, 200' may comprise one or more cooperating alignment features 250 (see Figs. 4A and 5A). The one or more alignment features 128 are adapted to engage with the one or more cooperating alignment features 250. For example, the alignment features 128 may be protrusions, and the cooperating alignment features 250 of the control unit 200, 200' may be slits, such as slits extending along the first body portion axis AxB. Alternatively, the alignment features 128 may be slits, e.g. extending along the channel axis AxC, and the cooperating alignment features 250 may be protrusions. The one or more cooperating alignment features 250 may be provided between the intermediate position 254 and the distal position 256 along the first body portion axis AxB.

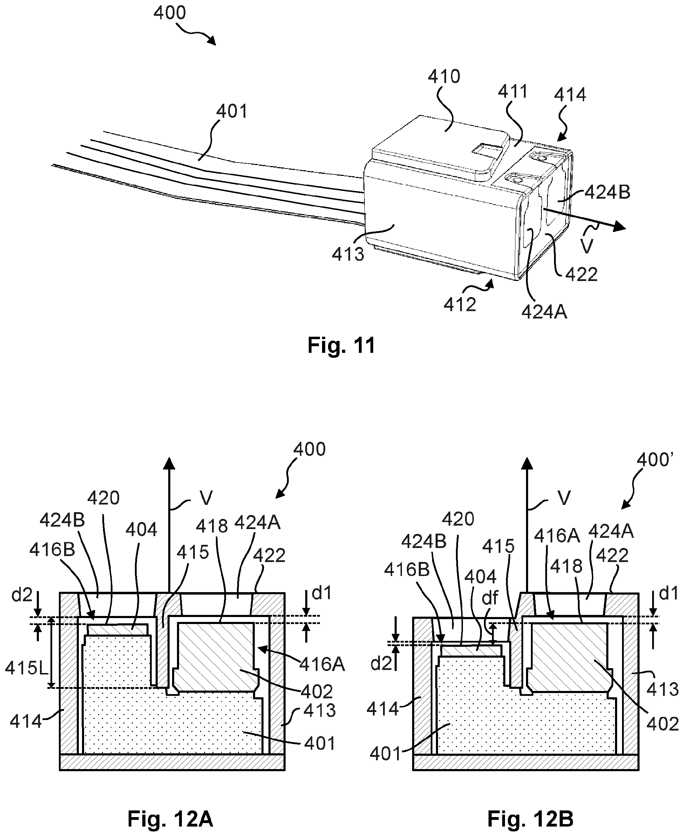

The blade unit 100 comprises a camera unit 400 comprising an image sensor 402 and optionally a light emitter 404. For example, a camera unit 400 as described in relation to Figs. 11-12 may be arranged in the distal part 106. The blade unit 100 comprises an electrical blade connector 112. The electrical blade connector 112 may comprise one or more terminals electrically connected to the image sensor 402 and/or the light emitter 404, e.g. via an electrical camera connector 401 extending from the electrical blade connector 112 to the camera unit 400 in the distal part 106. The electrical blade connector 112 is adapted to electrically connect the image sensor 402 and/or the light emitter 404 with the control unit 200, 200'. The electrical blade connector 112 is arranged in the channel 116, e.g., as illustrated, at a distal end 117 of the channel 116 opposite the proximal end 102 of the blade unit 100. The electrical blade connector 112 is adapted to connect with the electrical control unit connector 220 of the control unit 200, 200' (see Figs. 4A, 4B, 5A and 5B).

Fig. 6 schematically illustrates an exemplary laryngoscope 6 formed collectively of the control unit 200, as described in more detail in relation to Figs. 4A and 4B, and the blade unit 100, as described in more detail in relation to Fig. 3A. Similarly, Fig. 7 schematically illustrates another exemplary laryngoscope 6' formed collectively of the control unit 200' as described in more detail in relation to Figs. 5A and 5B, and the blade unit 100, as described in more detail in relation to Fig. 3A. As will be appreciated in the following, the laryngoscopes 6 and 6' comprises identical or very similar features. Thus, in the following Figs. 6 and 7 will be described together.

The control unit 200, 200' and the blade unit 100 collectively form the laryngoscope 6, 6' with the handle portion 110 extending along a first axis Ax1. A forward direction Fw of the laryngoscope 6, 6' is perpendicular to the first axis Ax1 in a direction towards the distal end 104 of the blade unit 100. A rearward direction Rw of the laryngoscope 6, 6' is opposite the forward direction Fw. The forward direction Fw and the rearward direction Rw are extending in a laryngoscope mid-plane defined by the first axis and the distal end 104 of the blade unit 100. In Fig. 6 the laryngoscope mid-plane is in the plane of the paper.

The laryngoscope 6, 6' comprises a camera unit 400 comprising an image sensor 402 and optionally a light emitter 404. As mentioned previously, in some examples the camera unit 400 may form part of the blade unit 100, while in others the camera unit 400 may form part of the control unit 200, 200'. The image sensor 402 is adapted to generate image data indicative of a view from the distal part 106 of the blade unit 100 in a viewing direction V at least partly towards the distal end 104. Preferably, the distal end 104 of the blade unit 100 is visible in the field of view, for the operator to see where, relative to the anatomy and/or to an intubation tube, the distal end 104 is positioned. However, it is preferred that the distal end 104 of the blade unit 100 does not take up excessive amount of the field of view. The control unit 200, 200' is adapted to receive the image data from the image sensor 402 as the image data is being generated by the image sensor 402. The camera unit 400, such as the image sensor 402 of the camera unit 400, may be arranged opposite the first surface 108 of the distal part 106. The camera unit 400, such as the image sensor 402 of the camera unit 400, may be arranged with a blade tip distance db between the front surface of the image sensor 402 and the distal end 104 of the blade unit 100. For example, the blade tip distance db may be between 19-50 mm.

The attachment part 204 of the control unit 200, 200' is positioned in the rearward direction Rw relative to the first axis Ax1. Furthermore, the control button 206 with the button surface 208 is positioned in the forward direction Fw relative to the attachment part 204. Thereby, the control button 206 may be operated by an index finger, when holding handle portion 110 of the laryngoscope 6, 6'. Providing the control button 206 to be operated by an index finger has the advantage that a firm grip of the handle portion by the thumb and the middle finger, ring finger and/or little finger may be maintained. It is a further advantage, of the control unit 200 of Fig. 6 that the control button 206 is provided such that operating the control button 206 does not interfere with a view of the control unit display.

The button surface 208 of the control button 206 has a button surface normal 208n. In some examples the button surface 208 may be curved in various directions. In such examples, the button surface normal 208n may be the normal at a center of the button surface 208. The button may be tilted in a forward direction, e.g. to be more easily accessible with the index finger. Hence, an angle between the button surface normal 208n and the forward direction Fw may be less than 90 degrees, such as less than 80 degrees. An angle between the button surface normal 208n and the first axis Ax1 may be between 0-180 degrees, such as between 1-179 degrees. The angle between the button surface normal 208n and the first axis Ax1 may be less than 90 degrees.

The control unit 200' comprises a connector cable 216 adapted to connect the control unit 200'to a monitor device, as illustrated in Fig. 2. The connector cable advantageously extends from the second body portion 202B, such as the attachment part 204 of the second body portion 202B, in a direction 217 substantially parallel to the first axis Ax1, as illustrated.

Fig. 8 schematically illustrates another exemplary laryngoscope assembly 4". The laryngoscope assembly 4" comprises a blade unit 100', which similarly to the blade unit 100 is adapted to be at least partly inserted into a patient's oral cavity, during an intubation procedure. The laryngoscope assembly 4" further comprises a control unit 200" with a control unit display 212. The control unit 200" is couplable to the blade unit 100'. The control unit 200" like the control unit 200, 200' as previously described comprises a body portion 202 having a first body portion 202A and a second body portion 202B. However, the first body portion 202A of the control unit 200" differs in that it comprises an extension portion 258, which may be a flexible element. The extension portion 258 extends to the distal end 203 of the first body portion 202A.

The camera unit 400, the image sensor 402 and/or the light emitter 404 may be arranged at the distal end 203 of the first body portion 202A. In this example, the blade unit 100' may be devoid of a camera unit, image sensor and/or light emitter, and instead comprise a channel to receive the extension portion 258 of the first body portion 202A of the control unit 200". Hence, when coupling the control unit 200" and the blade unit 100', the camera unit 400, image sensor 402 and/or light emitter 404 may be arranged at the distal part 106 of the blade unit 100', with a viewing direction at least partly towards the distal end 104 of the blade unit 100'. Such embodiment may be advantageous in that the blade unit 100' comprises less electronics, and therefore may be cheaper. However, on the other hand, the position of the camera unit 400 relative to the distal end of the blade unit 100' may be less precise and varying distances between the camera unit 400 and a window of the blade unit 100' may introduce unwanted reflections, which might influence image quality. Thus, while the blade unit 100 provided in, e.g., Figs. 3A-3D, wherein the camera unit 400 is provided as part of the blade unit, may be more expensive, it may still be preferred based on superior image quality. In both situations, the blade unit 100, 100' may be a single use, disposable, element.

Apart from the first body portion 202A comprising the extension portion 258 and elements thereof, the control unit 200" may be similar to the control unit 200 described previously. Furthermore, although not specifically illustrated, it should be appreciated that also the control unit 200' being connectable to the monitor device may comprise a first body portion 202A with an extension portion 258, and with the camera unit 400, the image sensor 402, and/or the light emitter 404 arranged at the distal end 203 of the first body portion 202A, as described, and be couplable to a blade unit 100' devoid of a camera unit, image sensor and/or light emitter.

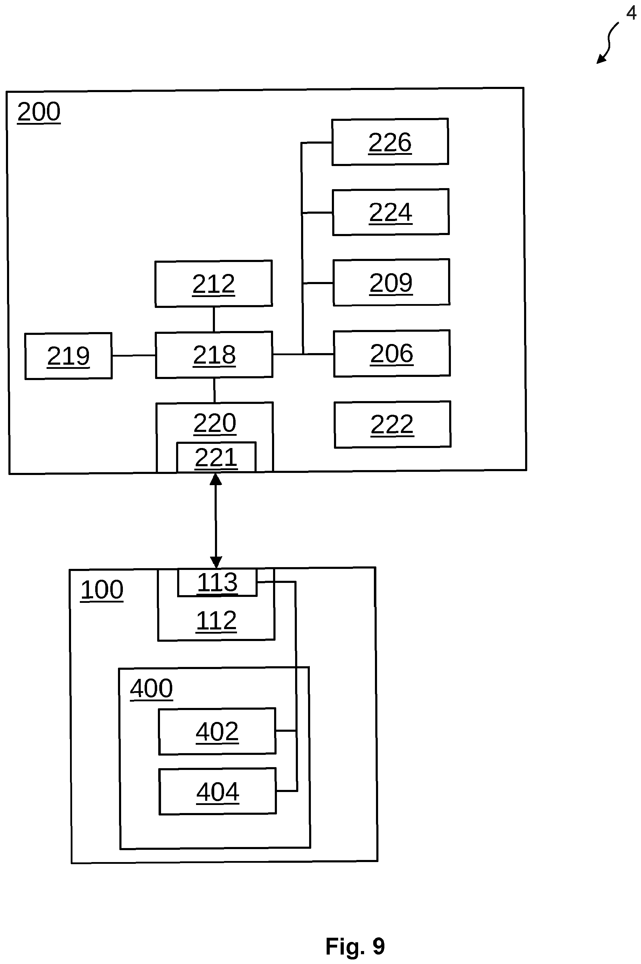

Fig. 9 is a block diagram schematically illustrating an exemplary laryngoscope assembly 4 comprising a control unit 200 and a blade unit 100. The laryngoscope assembly 4 may be the laryngoscope assembly 4 as exemplary illustrated, e.g., in Figs. 1 and 6.

The blade unit 100 comprises a camera unit 400 with an image sensor 402 and a light emitter 404. The image sensor 402 is positioned such that the image sensor 402 generates image data indicative of a view from a distal part of the blade unit in a direction at least partly towards the distal end. The light emitter 404 is positioned such that the light emitter 404 emits light along the viewing direction, such as to light up the field of view, or part thereof, of the image sensor 402.

The control unit 200, such as a control unit processing unit 218 of the control unit 200, is adapted to receive image data from the image sensor 402 as the image data is being generated by the image sensor 402. To achieve this, the control unit 200 is couplable to the blade unit 100. For example, as illustrated, the control unit 200 comprises an electrical control unit connector 220 with a plurality of terminals 221, and the blade unit 100 comprises an electrical blade connector 112 with a plurality of terminals 113. The electrical control unit connector 220 is couplable to the electrical blade connector 112, to electrically connect the terminals 221 of the electrical control unit connector 220 and the terminals 113 of the electrical blade connector 112. The terminals 113 of the electrical blade connector 112 may be electrically connected to the image sensor 402 and the light emitter 404 (e.g. via one or more not illustrated integrated circuitry). Thereby, when coupling the electrical control unit connector 220 and the electrical blade connector 112, the control unit 200, such as the control processing unit 218 of the control unit 200, establishes an electrical connection with the image sensor 402 and/or the light emitter 404 of the blade unit 100.

The control unit 200 may comprise a control unit display 212, and the control unit 200, such as the control processing unit 218 of the control unit, may be adapted to cause the control unit display 212 to display a live representation of the image data received from the image sensor 402.

The control unit 200 comprises a battery 222. The battery 222 may be provided as part of a battery pack, e.g. comprising mechanical elements and/or additional circuitry, e.g. for charging and/or determining level of charge of the battery 222. The battery 222 may be a rechargeable battery. The battery 222 may be adapted to power the control unit 200, such as electronic components of the control unit 200. The battery 222 may further be adapted to power the image sensor 402 and/or the light emitter 404. For example, the battery 222 may be adapted to power electronics of the blade unit 100, such as the image sensor 402 and the light emitter 404 of the blade unit 100, when the control unit 200 and the blade unit is coupled. For example, the battery 222 may be adapted to power electronics of the blade unit 100, such as the image sensor 402 and the light emitter 404 of the blade unit 100, via the terminals 113, 221 of the electrical connectors 112, 220.

The control unit 200 may comprise a power button 224 and/or a battery light indicator 226. The battery light indicator 226 may form part of the power button 224, e.g. the power button 224 may comprise the battery light indicator 226. For example, the battery light indicator 226 may be integrated in the power button 224. The power button 224 may be used to turn on/off the power of the control unit 200, and/or components thereof. For example, the power button 224 may be used to supply power to the image sensor 402 and/or the light emitter 404, and/or to the control unit display 212 and/or the control unit processing unit 218. The power button 224 may alternatively or additionally be used to check a current status of the battery 222. For example, the control unit 200, such as the control unit processing unit 218 of the control unit 200, may be adapted to detect a first user input corresponding to a first press (e.g. a short press) of the power button 224, and in accordance with detection of the first user input provide a battery status indication, e.g. with the battery light indicator 226 or with the control unit display 212. The control unit 200, such as the control unit processing unit 218 of the control unit 200, may further be adapted to detect a second user input corresponding to a second press (e.g. different from the first press, e.g. a long press) of the power button 224, and in accordance with detection of the second user input turn on/off the control unit 200. Thus, for example, the user may utilize a short press to check the battery status (e.g. without needing to turn on the control unit 200 for use) and may use a long press to turn on the control unit 200 for use. Differentiation between a long press and a short press may be done, e.g. by the control unit processing unit 218, based on the duration of the press, i.e. the press of the power button 224 may have a duration, which for a long press is more than or equal to a threshold amount of time (e.g. 1 second), while for a short press the duration is less than the threshold amount of time. The battery status indication, e.g. in accordance with detection of the first user input, may have a duration between 0.5-2.0 seconds.

The control unit 200, e.g. the control unit processing unit 218 or a dedicated circuitry of the control unit 200, may be adapted to determine a remaining battery capacity of the battery 222. In accordance with the remaining battery capacity being less than a low battery threshold (e.g. 20%), the battery status indication may comprise cyclically lighting the battery light indicator 226 a plurality of times with a first color (e.g. orange). In accordance with the remaining battery capacity being more than the low battery threshold and less than a high battery threshold (e.g. 50%) the battery status indication may comprise lighting the battery light indicator 226 once with the first color. In accordance with the remaining battery capacity being more than the high battery threshold the battery status indication may comprise lighting the battery light indicator 226 once with a second color (e.g. green).

The control unit 200, such as the control unit processing unit 218, may be adapted to activate a power saving mode, when the control unit 200 is not being used, or has not been used for a predetermined amount of time. For example, the control unit 200 may comprise an accelerometer, and the control unit 200, such as the control unit processing unit 218, may be adapted to activate the power saving mode, based on the accelerometer reading, which may be considered indicative of whether the device is being handled, i.e. is in use, or is being stored. The accelerometer reading may be combined with other variable, such as whether a blade is coupled or not, to decide if and/or when the power saving mode should be activated. For example, the power saving mode may be activated when an activity threshold, e.g. based on the accelerometer reading, has not been achieved. The activity threshold may be different depending on whether a blade unit is coupled to the control unit, e.g. when a blade unit is not coupled the activity threshold may be higher than if a blade unit is coupled to the control unit. In the power saving mode some functionalities may be turned off, e.g. disablement of touch-functionality of the control unit display, reduction of the screen brightness, etc.

The control unit 200 may comprise a control button 206, such as exemplified in Figs. 4A-4B. The control unit 200 may be adapted to execute functions, such as storing still images and/or video, in response to receipt of various user inputs, e.g. short press or long press, of the control button 206. The control button 206 may be coupled to the control unit processing unit 218, and the control unit processing unit 218 may be used to interpret user input received by the control button 206 and/or to execute actions in accordance with the received user input. The control unit 200, such as the control processing unit 218, may be adapted to distinguish between various types of user input at the control button 206. For example, the control unit 200, such as the control processing unit 218, may differentiate between a short press, e.g. having a duration shorter than a threshold amount of time (e.g. 1 second) and a long press, e.g. having a duration longer than the threshold amount of time.

For example, the control unit 200, such as the control unit processing unit 218, may be adapted to detect a first user input corresponding to a first press of the control button 206 (e.g. a short press), and in accordance with detection of the first user input cause recording (e.g. in memory 219 of the control unit 200) of a first image file corresponding to the image data received when the first user input was detected. Similarly, the control unit 200, such as the control unit processing unit 218, may be adapted to detect a second user input corresponding to a second press of the control button 206 (e.g. a long press), and in accordance with detection of the second user input cause initiating a recording (e.g. in memory 219 of the control unit 200) of a first video file corresponding to the image data received after detection of the second user input. After detection of the second user input, the control unit 200, such as the control unit processing unit 218, may be adapted to detect a third user input corresponding to the second press of the control button 206 (e.g. a long press), and in accordance with detection of the third user input cause ending the recording of the first video file. Thus, the first video file may correspond to the image data received between detection of the second user input and the third user input. Thus, for example, a short press may be used to capture a still image, while a long press may be used to start and stop recording of a video. During recording of a video file, i.e. between detection of the second user input ant the third user input, it may be possible, e.g. by a short press, to also capture still images, i.e. corresponding to individual images of the video file. When capturing a still image and/or when starting or stopping recording of a video, the control unit 200, such as the control unit processing unit 218, may cause the control unit display 212 to briefly show a colored border, e.g. around the live image, to indicate to the user that a video and/or a still image has been recorded. In some examples, during video recording, the border may be continuously shown while recording, and disappear when recording is stopped. In some examples, the control unit 200, such as the control unit processing unit 218, may be adapted to start recording of a video file in response to detection of the control unit 200 being coupled to a blade unit. Thereby, the entire procedure, from assembling the laryngoscope may be recorded.

The control unit 200, such as the control unit processing unit 218, may be adapted to automatically stop a video recording if it is detected that the remaining memory is below a certain threshold. Thereby, a complete video file may be stored in the memory 219, before the memory 219 is completely full, decreasing the risk of storing potentially corrupt files. In some examples, the user interface on the control unit display 212 may show remaining memory, in terms of how many minutes of video recording can be stored.

The electrical control unit connector 220 may also be used for connecting a data transfer cable to the control unit 200, e.g. to allow transfer of data, e.g. image and/or video data, from the memory 219 of the control unit 200 to an external device, such as a laptop, a smart phone, a tablet, etc. The data transfer cable may have a plug at one end resembling the electrical blade connector 112 and a USB plug at the other end, thereby facilitating connection between the control unit 200 and an external device with a USB port. When being connected to an external device the control unit 200 may act like a flash drive. When being connected to an external device, the control unit display may display "file transfer" to indicate that files are being transferred to the external device. In some examples, when being connected to an external device, the file transfer capability may be powered by the external device. In some examples, the battery of the control unit 200 may not be charged but may not be drained either. In some examples, after decoupling the data transfer cable, the control unit 200 returns to the same state (on/off) as it was in before the data transfer cable was connected.

The control unit 200 may comprise a control button light indicator 209, which, as mentioned in relation to Figs. 4A and 4B may be integrated with the control button 206. The control button light indicator 209 may be used to indicate various information associated with the control unit 200, such as, for example, whether a blade unit 100 is coupled or not. For example, the control unit 200, such as the control unit processing unit 218, may detect coupling with the blade unit 100, and in accordance with detection of coupling between the blade unit 100 and the control unit 200 provide a first visual indication with the control button light indicator 209. In accordance with detection of no coupling between the blade unit 100 and the control unit 200, the control unit 200, such as the control unit processing unit 218, may provide a second visual indication with the control button light indicator. For example, the first visual indication may correspond to the control button light indicator 209 being steadily lit, and the second visual indication may correspond to the control button light indicator 209 blinking. Alternatively or additionally, the first visual indication may be with a first color and the second visual indication may be with a second (different) color.

It should be appreciated that the description of Fig. 9 applies mutatis mutandis to a laryngoscope assembly 4" of Fig. 8. In such example, the camera unit 400 may form part of the control unit 200 (corresponding to control unit 200" of Fig. 8), and at least the electronic elements of the electrical connectors 220, 112 may be omitted. In such system, the blade unit 100 may be devoid of electronic elements.

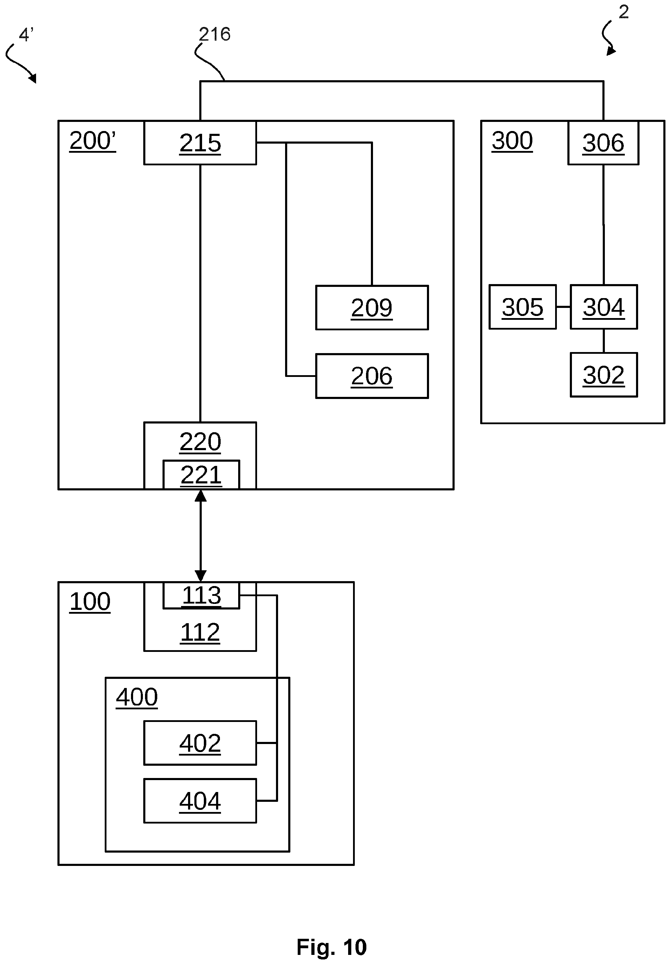

Fig. 10 is a block diagram schematically illustrating an exemplary visualization system 2 with a laryngoscope assembly 4' and a monitor device 300. The laryngoscope assembly 4' comprises a control unit 200' and a blade unit 100. The laryngoscope assembly 4' may be the laryngoscope assembly 4' as exemplary illustrated, e.g., in Figs. 2 and 7. The control unit 200' may be connectable to a monitor device. The blade unit 100 may be similar to the blade unit 100 as illustrated and explained in relation to Fig. 9. The monitor device 300 may be the monitor device 300 as illustrated in Fig. 2. For example, the monitor device 300 may comprise a monitor display 302, a monitor processing unit 304 and a monitor connector 306.

The control unit 200' is adapted to receive image data from the image sensor 402 as the image data is being generated by the image sensor 402. To achieve this, the control unit 200' is couplable to the blade unit 100. For example, as illustrated, the control unit 200' comprises an electrical control unit connector 220 with a plurality of terminals 221, and the blade unit 100 comprises an electrical blade connector 112 with a plurality of terminals 113. The electrical control unit connector 220 is couplable to the electrical blade connector 112, to electrically connect the terminals 221 of the electrical control unit connector 220 and the terminals 113 of the electrical blade connector 112. The terminals 113 of the electrical blade connector 112 may be electrically connected to the image sensor 402 and the light emitter 404 (e.g. via one or more not illustrated integrated circuitry). Thereby, when coupling the electrical control unit connector 220 and the electrical blade connector 112, the control unit 200' establishes an electrical connection with the image sensor 402 and/or the light emitter 404 of the blade unit 100.

The control unit 200' may comprise a connector cable 216. The connector cable 216 may be adapted to connect the control unit 200' to the monitor device 300. For example, the connector cable 216 may be connectable to a monitor connector 306 of the monitor device 300. In some examples, the connector cable 216 may have a plug and the monitor connector 306 may have a corresponding socket for receiving the plug of the connector cable 216. The control unit 200' may comprise a cable interface 215 connecting the connector cable 216 to the internal electronics of the control unit 200'. The connector cable 216 may be decouplable from the remainder of the control unit 200' at the cable interface 215 or it may be fixedly attached to the remainder of the control unit 200'. The control unit 200', such as the control processing unit 218 of the control unit 200', may be adapted to, e.g. via the connector cable 216, cause the monitor display 302 of the monitor device 300 to display a live representation of the image data received from the image sensor 402.

In some examples, the control unit 200' may comprise a control unit processing unit 218, as similar described in Fig. 9, e.g. for receiving and handling the image data from the image sensor 402. In such example, the control processing unit 218 may forward the image data to the monitor processing unit 304 of the monitor device 300 via the connector cable 216. However, as illustrated, the control unit 200' may, alternatively, omit a control unit processing unit and provide a connection between the connector cable 216 and the electrical control unit connector 220, such that effectively the control unit 200' provides a connection between the monitor device 300 and the blade unit 100. Thereby, the monitor processing unit 304 may display the live representation by the monitor display 302.

Compared to the control unit 200, the control unit 200' may omit a battery, and may be powered by the monitor device 300, e.g. through the connector cable 216. The monitor device 300 may comprise a battery and/or a power supply to power both the monitor device 300 and the control unit 200'. However in some examples, the control unit 200' may comprises a battery 222 as explained in relation to Fig. 9. The control unit 200' may be adapted to power the image sensor 402 and/or the light emitter 404. For example, the control unit 200' may be adapted to power electronics of the blade unit 100, such as the image sensor 402 and the light emitter 404 of the blade unit 100, when the control unit 200' and the blade unit 100 is coupled. For example, the control unit 200 may be adapted to power electronics of the blade unit 100, such as the image sensor 402 and the light emitter 404 of the blade unit 100, via the terminals 113, 221 of the electrical connectors 112, 220.

Compared to the control unit 200, the control unit 200' may omit a power button and/or a battery light indicator, e.g. as the control unit 200' may be powered by the monitor device 300, and therefore does not need a power on/off capability and/or an indicator of the battery status. However, the control unit 200' may in other examples comprise a power button 224 and/or a battery light indicator 226 as explained in relation to Fig. 9.

Although the control unit 200' in the examples is devoid of a display, also a control unit comprising both a connector cable 216 and a display unit may be realized, based on the present disclosure. Thus, the features of the control units 200 200', 200" may be combinable.

The control unit 200' may comprise a control button 206, such as exemplified in Figs. 5A-5B. The control unit 200' may be adapted to cause functions, such as storing still images and/or video, in response to receipt of various user inputs, e.g. short press or long press, of the control button 206. In cases where the control unit 200' would comprise a control processing unit, the control button 206 may be coupled to the control unit processing unit 218, and function as described in relation to Fig. 9. However, alternatively, and as illustrated, the control button 206 may be connected to the connector cable 216, and the monitor device 300, such as the monitor processing unit 304 may be used to interpret user input received by the control button 206 and/or to execute actions in accordance with the received user input. The monitor device 300, such as the monitor processing unit 304, may be adapted to distinguish between various types of user input at the control button 206. For example, the monitor device 300, such as the monitor processing unit 304, may differentiate between a short press, e.g. having a duration shorter than a threshold amount of time (e.g. 1 second) and a long press, e.g. having a duration longer than the threshold amount of time.

For example, the monitor device 300, such as the monitor processing unit 304, may be adapted to detect a first user input corresponding to a first press of the control button 206 (e.g. a short press), and in accordance with detection of the first user input cause recording (e.g. in memory 305 of the monitor device 300) of a first image file corresponding to the image data received when the first user input was detected. Similarly, the monitor device 300, such as the monitor processing unit 304, may be adapted to detect a second user input corresponding to a second press of the control button 206 (e.g. a long press), and in accordance with detection of the second user input cause initiating a recording (e.g. in memory 305 of the monitor device 300) of a first video file corresponding to the image data received after detection of the second user input. After detection of the second user input, the monitor device 300, such as the monitor processing unit 304, may be adapted to detect a third user input corresponding to the second press of the control button 206 (e.g. a long press), and in accordance with detection of the third user input cause ending the recording of the first video file. Thus, the first video file may correspond to the image data received between detection of the second user input and the third user input. Thus, for example, a short press may be used to capture a still image, while a long press may be used to start and stop recording of a video. During recording of a video file, i.e. between detection of the second user input ant the third user input, it may be possible, e.g. by a short press, to also capture still images, i.e. corresponding to individual images of the video file. When capturing a still image and/or when starting or stopping recording of a video, the monitor device 300, such as the monitor processing unit 304, may cause the monitor device display 302 to briefly show a colored border, e.g. around the live image, to indicate to the user that a video and/or a still image has been recorded. In some examples, during video recording, the border may be continuously shown while recording, and disappear when recording is stopped. In some examples, the monitor device 300, such as the monitor processing unit 304, may be adapted to start recording of a video file in response to detection of the control unit 200' being coupled to a blade unit. Thereby, the entire procedure, from assembling the laryngoscope may be recorded. In case the control unit 200' is coupled to a blade unit prior to connecting the control unit 200'to the monitor device 300, the monitor device 300, such as the monitor processing unit 304 may be adapted to start recording in response to connecting the control unit 200' to the monitor device 300.

The monitor device 300, such as the monitor processing unit 304, may be adapted to automatically stop a video recording if it is detected that the remaining memory is below a certain threshold. Thereby, a complete video file may be stored in the memory 305 before the memory 305 is completely full, decreasing the risk of storing potentially corrupt files. In some examples, the user interface on the monitor device display 302 may show remaining memory, in terms of how many minutes of video recording can be stored.

The control unit 200' may comprise a control button light indicator 209, which, as mentioned in relation to Figs. 5A and 5B may be integrated with the control button 206. The control button light indicator 209 may be used to indicate various information associated with the control unit 200', such as, for example, whether a blade unit 100 is coupled or not. For example, the control unit 200' and/or the monitor device 300, such as the monitor processing unit 304, may detect coupling with the blade unit 100, and in accordance with detection of coupling between the blade unit 100 and the control unit 200' provide a first visual indication with the control button light indicator 209. In accordance with detection of no coupling between the blade unit 100 and the control unit 200, a second visual indication may be provided with the control button light indicator 209. For example, the first visual indication may correspond to the control button light indicator 209 being steadily lit, and the second visual indication may correspond to the control button light indicator 209 blinking. Alternatively or additionally, the first visual indication may be with a first color and the second visual indication may be with a second (different) color.

Like for Fig. 9, it should be appreciated that the description of Fig. 10 applies mutatis mutandis to a laryngoscope assembly comprising the control unit 200' being connectable to the monitor device modified to comprise the extension portion 258, as described with respect to Fig. 8. In such example, the camera unit 400 may form part of the control unit 200', and at least the electronic elements of the electrical connectors 220, 112 may be omitted. In such system, the blade unit 100 may be devoid of electronic elements.

Fig. 11 schematically illustrates an exemplary camera unit 400, which may be provided at the distal part 106 of the blade unit 100, as described in relation to Fig. 3A or at a distal end 203 of the control unit 200", as described in relation to Fig. 8. The camera unit 400 comprises an image sensor 402 and a light emitter 404 (see Figs. 12A and 12B). The camera unit 400 comprises an electrical camera connector 401, which, for example, may electrically connect the camera unit 400, such as the image sensor 402 and/or the light emitter 404 thereof, to the electrical blade connector 112 of the blade unit 100, and/or to the control processing unit 218 of the control unit 200 (see, e.g., Figs. 9 and 10 and the related description). The electrical camera connector 401 may be a flexible printed circuit. Alternatively, it may be provided by a collection of wires or other techniques known in the art.

Fig. 12A schematically illustrates a cross-sectional view of the camera unit 400 of Fig. 11. Fig. 12B schematically illustrates a cross-sectional view of an exemplary camera unit 400', which resembles the camera unit 400, although having the image sensor 402 and the light emitter 404 being (more) offset along the viewing direction. For convenience, Figs. 11, 12A and 12B, will be described together in the following.

The camera unit 400 comprises a camera unit housing 410. The camera unit housing comprises a first side wall 411 and a second side wall 412. The camera unit housing 410 further comprises a third side wall 413 extending between the first side wall 411 and the second side wall 412. The camera unit housing 410 further comprises a fourth side wall 414 extending between the first side wall 411 and the second side wall 412. The camera unit housing 410 further comprises a separating wall 415 extending between the first side wall 411 and the second side wall 412. The separating wall 415 is further arranged between the third side wall 413 and the fourth side wall 414. The separating wall 415 is acting to prevent or reduce light from the light emitter 420 travelling directly to the image sensor 402, which may distort the images generated by the image sensor 402. Thus, the separating wall 415 is preferably opaque to light, at least within the visible spectrum of light and optionally ultraviolet and/or infrared light.

A first housing compartment 416A of the camera unit housing 410 is defined by the first side wall 411, the second side wall 412, the third side wall 413 and the separating wall 415. The image sensor 402 is accommodated in the first housing compartment 416A. The image sensor is adapted to generate image data indicative of a view along a viewing direction V from a front surface 418 of the image sensor 402. A second housing compartment 416B of the camera unit housing 410 is defined by the first side wall 411, the second side wall 412, the fourth side wall 414 and the separating wall 415. The light emitter 404 is accommodated in the second housing compartment 416B. The light emitter 404 is adapted to emit light along the viewing direction V from a front surface 420 of the light emitter 404. The separating wall 415 may have a length 415L along the viewing direction V, e.g., of more than 1.5 mm, such as more than 2 mm. The viewing direction V corresponds to the viewing direction V of Figs. 6 and 7, when the camera unit 400 is arranged as intended in the blade unit 100, i.e., being from the distal part 106 of the blade unit in a direction at least partly towards the distal end 104 of the blade unit 100.

The camera unit housing 400 may further comprise a front side wall 422 extending between the first side wall 411 and the second side wall 412 and between the third side wall 413 and the fourth side wall 414. The front side wall has a first window 424A and a second window 424B. The separating wall 415 adjoins the front side wall 422 between the first window 424A and the second window 424B. The first window 424A provides an optical connection between the first housing compartment 416A through the front side wall 422. The second window 424B provides an optical connection between the second housing compartment 416B through the front side wall 422. Thereby, the image sensor 402 may "see" through the front side wall 422 via the first window 424A, and the light emitter 404 may emit light through the front side wall 422 via the second window 424B.

The image sensor 402 may be arranged with a distance d1 between the front surface 418 of the image sensor 402 and the first window 424A. The distance may reduce undesired reflections possibly distorting the images generated by the image sensor 402. The distance d1 between the front surface 418 of the image sensor 402 and the first window 424A may be between 0.1 mm and 0.5 mm, such as between 0.2 mm and 0.4 mm. The light emitter 404 may be arranged with a distance d2 between the front surface 420 of the light emitter 404 and the second window 424B. The distance d2 between the front surface 420 of the light emitter 404 and the second window 424B may be between 0.1 mm and 0.5 mm, such as between 0.2 mm and 0.4 mm. The distance d1 between the front surface 418 of the image sensor 402 and the first window 424A may be smaller than the distance d2 between the front surface 420 of the light emitter 404 and the second window 424B.

The image sensor 402 and the light emitter 404 may be arranged with an offset distance df along the viewing direction V, e.g. as illustrated in Fig. 12B, between the front surface 418 of the image sensor 402 and the front surface 420 of the light emitter 404. For example, the light emitter 404 may be arranged behind the image sensor 402. The offset distance df may be between 0.1 mm and 1.5 mm, such as between 0.5 mm and 1 mm. Alternatively, the image sensor 402 and light emitter 404 may be arranged flush, as illustrated in Fig. 12A. In the example of Fig. 12B illustrating the image sensor 402 and the light emitter 404 being arranged with the offset distance df, there may also be an offset distance between the windows 424A, 424B. However, alternatively, the front of the windows 424A, 424B may be flush like in Fig. 12A while the mage sensor 402 and the light emitter 404 are arranged with the offset distance df.

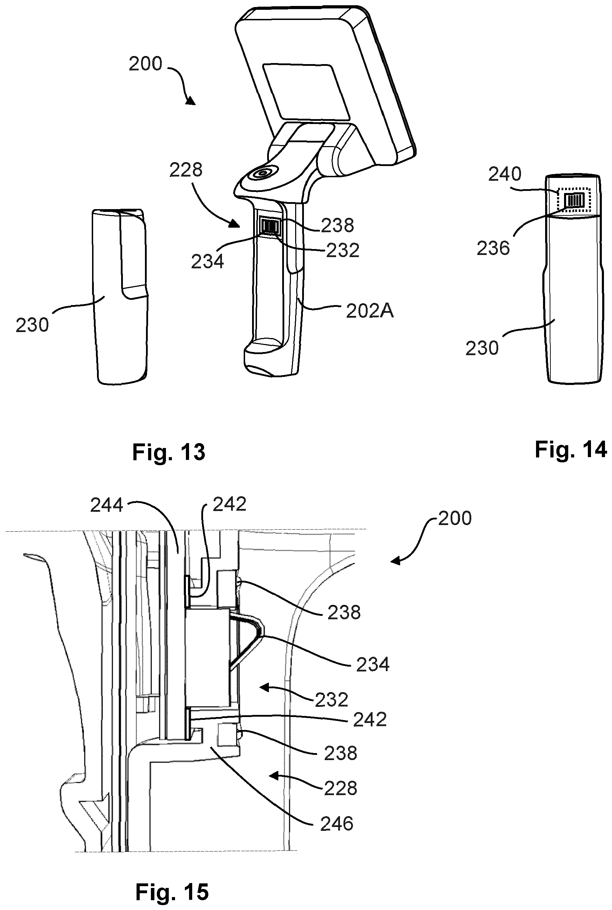

Fig. 13 schematically illustrates the exemplary control unit 200, and more particularly illustrates that the control unit 200 may utilize a replaceable battery pack 230 adapted to power the control unit 200. The control unit 200 comprises a battery receiver 228 adapted to receive the replaceable battery pack 230. Fig. 14 schematically illustrates the battery pack 230 from the side to be received by the battery receiver 228. The battery receiver 228 comprises a battery connector 232 with a plurality of battery connector terminals 234. The battery connector terminals 234 are adapted to cooperate with corresponding connection terminals 236 of the replaceable battery pack 230. The battery pack 230 may comprise a rechargeable battery. Thus, the battery pack 230 may be removed from the control unit 200 and be placed in a charger adapted to charge the battery.

The battery pack 230 may be dimension such that it is retained in the battery receiver 228 by friction. Hence, mechanical locking between the battery pack 230 and the battery receiver 228 may be omitted. This means that the battery pack 230 can easily be attached to the battery receiver 228 by just pushing the battery pack 230 into place. The battery pack 230 may provide parts which are slightly wider than the first body portion 202A, and which thereby protrudes slightly from the sides of the first body portion 202A. Thereby, removal of the battery pack 230 may be made easier.

The battery receiver 228 comprises an outer connector seal 238 encircling the battery connector 232. The outer connector seal 238 is adapted to contact a corresponding surface 240 of the replaceable battery pack 230, when the replaceable battery pack 230 is received in the battery receiver 228. The corresponding surface 240 of the replaceable battery pack 230 is a surface encircling the connection terminals 236 of the replaceable battery pack 230. Thereby, water or other liquid is restricted from entering the electrical interface between the battery pack 230 and the battery receiver 228. Hence, risk of liquid interfering the electrical connection is reduced. The replaceable battery pack 230 may be devoid of an outer sealing element adapted to contact a surface of the control unit 200, such as a surface of the battery receiver 228. Hence, the corresponding surface 240 of the replaceable battery pack 230 contacting the outer connector seal 238 may merely be a part of the outer surface of the battery pack, e.g. as indicated by the dotted line in Fig. 14, and may not be visually distinct from the remainder of such surface.

The battery receiver 228 may form part of the first body portion 202A of the control unit 200, as illustrated. As mentioned previously, the first body portion 202A may be adapted to be received and enclosed by the blade unit, such as the handle portion 110 of the blade unit 100 (see, e.g. Fig. 6). Thereby, the battery pack 230 and the battery receiver 228 may be fully enclosed by the blade unit 100, i.e. the battery pack 230 does not form an outer surface of the assembled laryngoscope, and the battery pack 230 is kept away from contact with the patient. As the battery pack 230 does not form part of the outer surface of the assembled laryngoscope, the battery pack is protected, which is particularly advantageous for a rechargeable battery pack 230.

Fig. 15 schematically illustrates, an exemplary cross-sectional view of a battery connector 232, such as the battery connector 232 of the battery receiver 228 as also described in relation to Fig. 13. As Figs 13-15 are schematic, minor variations may exist between the figures, and some details may be shown in one or two of the figures, while being omitted from others. As mentioned above, the battery receiver 228 comprises a battery connector 232 with a plurality of battery connector terminals 234 adapted to cooperate with corresponding connection terminals 236 of the replaceable battery pack 230 (see Fig. 14). The battery receiver 228 comprises an outer connector seal 238 encircling the battery connector 232 adapted to contact a corresponding surface 240 of the replaceable battery pack 230, when the replaceable battery pack 230 is received in the battery receiver 228.

The control unit 200 may comprise an internal sealing 242 encircling the battery connector 232. The internal sealing 242 prevents liquid from entering from the battery receiver 228 and into an internal space of the control unit 200 when the replaceable battery pack 230 is not received in the battery receiver 228. The control unit 200 may further comprise a battery connector circuitry 244, e.g. a printed circuit board or other electronic circuitry known in the art, used to couple the battery connector terminals 234 to other relevant electronic components of the control unit 200, and to communicate with electronic components, such as a battery fuel gauge, of the battery pack 230. The battery connector 232 is attached to a first side of the battery connector circuity 244. The internal sealing 242 is arranged between the battery connector circuitry 244 and a shell element 246 forming part of the battery receiver 228. Thereby, water or other liquid is restricted from entering into the internal compartments of the control unit 200 at the battery connector 232, even when the battery pack 230 is not received. Hence, risk of liquid interfering with and potentially damaging the electronic components inside the control unit 200 is reduced, also in cases where the battery pack 230 is not received in the battery receiver 228.

As mentioned above, in case the control unit 200', e.g., of Figs. 5A and 5B or the control unit 200", e.g., of Fig. 8, were to comprise a battery, the battery may be provided in a battery pack, such as the battery pack 230 as described, and the control unit 200', 200" may comprise a battery receiver, such as the battery receiver 228 as described above.

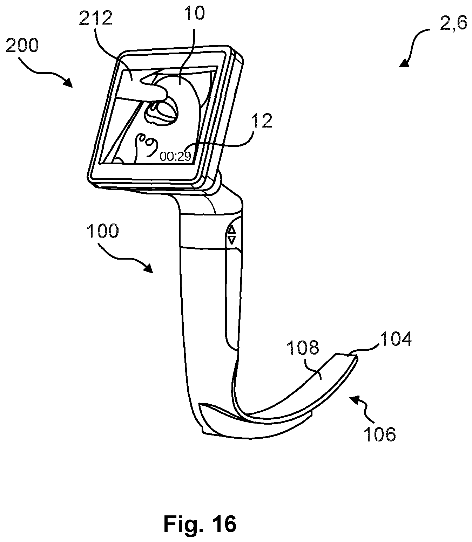

Fig. 16 schematically illustrates an exemplary laryngoscope system 2. The illustrated laryngoscope system 2 comprises a laryngoscope 6, such as the laryngoscope 6, as also described in relation to, e.g., Figs. 1A, 1B and Fig. 6. Although the following is described with reference to the illustrated laryngoscope 6, which is illustrated with a control unit 200 comprising a display 212, the functionality could be similarly implemented on another or complimentary laryngoscope system, e.g., wherein the laryngoscope is coupled to a monitor device with a display, such as illustrated in Fig. 2.

The laryngoscope system 2 comprises a blade unit 100, as also explained in relation to previous figures, extending from a proximal end 102 to a distal end 104, and having a distal part 106 extending to the distal end 104. The distal part 106 comprises a first surface 108. The distal part 106 is adapted to be inserted into a patient's oral cavity with the first surface 108 facing the patient's tongue. The laryngoscope system 2 further comprises an image sensor adapted to generate image data indicative of a view in a viewing direction from the distal part 106 of the blade unit 100 in a direction at least partly towards the distal end 104. The image sensor may be provided either as part of the blade unit 100 as described in relation to Figs. 3A-3D, or as part of the control unit, as described in relation to Fig. 8.

The laryngoscope system 2 comprises a display 212, which, as illustrated may be a display 212 forming part of the control unit 200. However, the display may alternatively be a display of a monitor device to which the laryngoscope 6 is connected.

The laryngoscope system 2, such as the control unit 200 (e.g. the control processing unit 212 of the control unit 200, cf. Fig. 9), is adapted to display a live representation 10 of the image data with the display 212. The laryngoscope system 2, such as the control unit 200 (e.g. the control processing unit 212), may further be adapted to detect a trigger-event, and in response to detection of the trigger event: start a timer function, and display, concurrently with the live representation 10, a value of the timer function 12 with the display 212.

Some guidelines specify that an operator should revert to alternative methods for ensuring ventilation of the patient, if intubation has not been completed within a predefined amount of time, e.g. within 30 seconds. Thus, with the mentioned functionality, the operator is provided information of the current situation and is notified when having to revert to other options. Thus, observance of guidelines may be assisted. In some examples, the timer function may be a countdown decreasing towards zero. Such countdown may start from a pre-defined amount of time, e.g. according to a guideline's predefined amount of time before reverting to alternative methods. The pre-defined amount of time may, for example, be 30 seconds. In other examples, the timer function may be a timer starting from zero and counting upwards.

The laryngoscope system 2 may detect coupling between the control unit 200 and the blade unit 100, and the trigger event may correspond to coupling of the blade unit 100 and the control unit 200. Thus, the timer function may start once the operator couples the blade unit 100 and the control unit 200. Alternatively, the trigger event may correspond to a user input, e.g. a press of a button or a touch sensitive display of the control unit 200. Alternatively, the laryngoscope system 2, such as the control unit 200 (e.g. the control processing unit 212 of the control unit 200, cf. Fig. 9), may detect, e.g. by image analysis of the image data, insertion of the distal part of the blade unit into a patient's oral cavity. Hence, the trigger event may correspond to insertion of the distal part of the blade unit into a patient's oral cavity, e.g. as detected by image analysis of the image data.

The operator may be visually notified when he/she approaches a limit, e.g. the limit as set by the guideline. Hence, after start of the timer function, in accordance with the value of the timer function being outside a low-threshold time range (e.g. 5 seconds), the laryngoscope system may display the value of the timer function 12 having a first visual characteristic, and in accordance with the value of the timer function being within the low-threshold time range, the laryngoscope system may display the value of the timer function 12 having a second visual characteristic different from the first visual characteristic. For example, the second visual characteristic may be a different color, e.g. orange, and/or the second visual characteristic may be blinking.

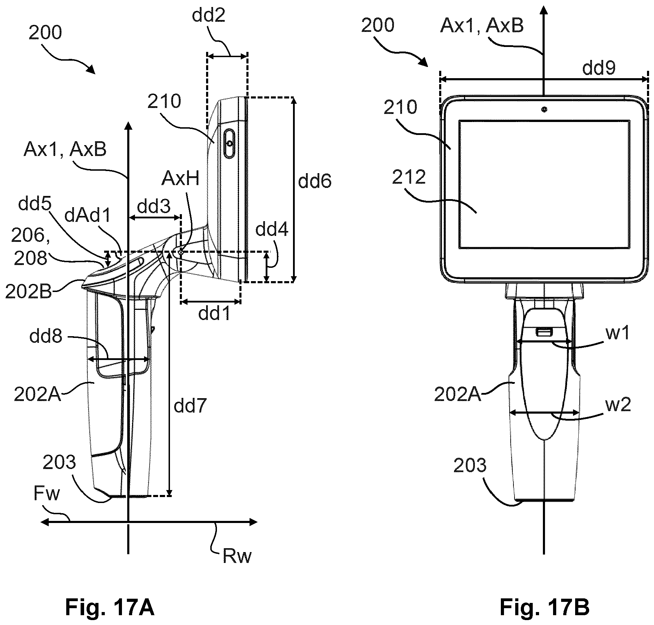

Figs. 17A and 17B show from the side and the front, respectively, an exemplary control unit 200, such as the control unit 200 as described in relation to previous figures (e.g. in Figs. 4A and 4B).

As seen, the control unit display element 210 may be positioned more towards the user, i.e. in the rearward direction Rw relative to the first axis Ax1 and/or the body portion axis AxB following the terminology as explained in relation to Figs. 4A, 4B and 6. The inventors, have found, and verified by user tests, that having the display shifted towards the user facilitates a superior weight distribution of the control unit 200, on its own as well as when being coupled with the blade unit. Thereby, the control unit 200 may facilitate better handling, and consequently improve intubation.

The control unit 200 has a first control unit distance dd1 between the control unit display 212 and the hinge axis AxH. The first control unit distance dd1 may be between 20-30 mm, such as between 23-25 mm.

The control unit display element 210 has a second control unit distance dd2 between a rearward surface of the control unit display element 210 and a forward surface of the control unit display element 210, e.g. the control unit display 212. The second control unit distance dd2 may be between 10-20 mm, such as between 15-18 mm.

The hinge axis AxH may be positioned with a third control unit distance dd3 from the first axis Ax1 and/or the body portion axis AxB. The third control unit distance dd3 may be between 20-30 mm, such as between 25-29 mm.

The hinge axis AxH may be positioned, along the first axis Ax1 and/or body portion axis AxB, between a top edge and a bottom edge of the control unit display element 210. The hinge axis AxH may be positioned with a fourth control unit distance dd4 from the bottom edge of the control unit display element 210. The fourth control unit distance dd4 may be between 10-15 mm, such as between 12-13 mm.

An upper surface of the second body portion 202B may be inclined and the button surface 208 of the control button 206 may be provided below the hinge axis AxH, along the first axis Ax1 and/or the body portion axis AxB, by a fifth control unit distance dd5. The fifth control unit distance dd5 may be between 1-10 mm, such as between 4-6 mm. The button surface 208 and/or the upper surface of the second body portion 202B may form upper body portion angle dAd1 with respect to a line perpendicular to the first axis Ax1 and/or the body portion axis AxB. The upper body portion angle dAd1 may be between 20-35 degrees, such as between 25-30 degrees.

The control unit display element 210 may have a sixth control unit distance dd6, i.e. a height, between the bottom edge and the top edge of the control unit display element 210. The sixth control unit distance dd6 may be between 60-100 mm, such as between 75-85 mm.

The control unit 200 may have a seventh control unit distance dd7, along the first axis Ax1 and/or the body portion axis AxB, between the distal end 203 of the first body portion 202A and the hinge axis AxH. The seventh control unit distance dd7 may be between 90-110 mm, such as between 95-105 mm. Although not illustrated the control unit 200 may have a distance between the button surface 208 and the distal end 203 of the first body portion 202A, which may be between 85-105 mm, such as between 90-100 mm.

The first body portion 202A may have an eighth control unit distance dd8 between a front surface facing the forward direction Fw and a rear surface facing the rearward direction Rw. The front surface may be a surface of a replaceable battery pack. The eighth control unit distance dd8 may be perpendicular to the first axis Ax1 and/or the body portion axis AxB. The eighth control unit distance dd8 may be between 20-35 mm, such as between 25-30 mm.

The control unit display element 210 may have a ninth control unit distance dd9 between a left edge and a right edge of the control unit display element 210. The ninth control unit distance dd9 may be between 75-100 mm, such as between 80-90 mm.

As also described in relation to Fig. 4B, the first body portion 202A may have a first width w1 at a first position and the first body portion may have a second width w2 at a second position. The second width w2 may be greater than the first width w1. The first width w1 may be between 25-35 mm, such as between 26-29 mm. The second width w2 may be between 25-35 mm, such as between 29-32 mm.

The width of the first body portion 202A, e.g. the second width w2, and/or the eighth control unit distance dd8 may decrease towards the distal end 203. The width of the first body portion 202A and/or the eighth control unit distance dd8 may decrease to facilitate easier insertion of the first body portion 202A in the channel of the blade unit.

The inventors have found that the geometry and size of the exemplary control unit 200 are advantageous in terms of ergonomics and handling.

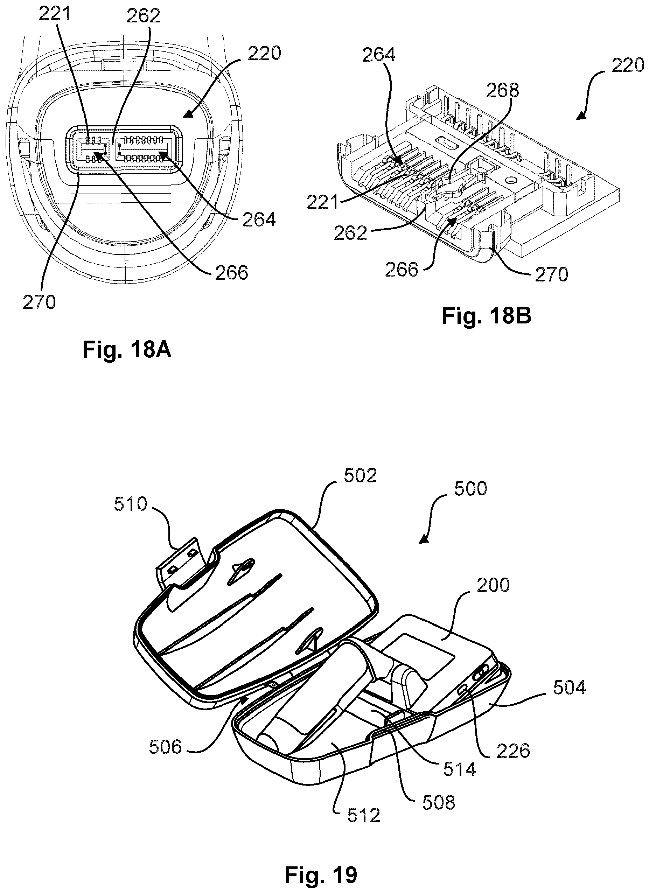

Figs. 18A and 18B show an exemplary electrical control unit connector 220 of the control unit 200, 200'. The electrical control unit connector 220 is adapted to electrically connect the control unit 200, 200' to the camera unit 400, image sensor 402 and/or light emitter 404 of the blade unit 100, as explained previously. Fig. 18B shows a cut open view of the electrical control unit connector 220. As seen, the electrical control unit connector 220 may comprise an alignment feature 262, e.g. a wall, separating the electrical control unit connector 220 in two unequal parts 264, 266. Furthermore, a retention element 268, e.g. a spring, may be provided in combination with the separating element 262. The blade unit 100, as illustrated in Fig. 3D, comprises a corresponding electrical blade connector 112. The electrical blade connector 112 is sectioned into two unequal parts corresponding to the parts 264, 266 of the electrical control unit connector 220. Furthermore, the electrical blade connector 112 of the blade unit 100 may comprise indents for cooperation with the retention element 268.

The electrical control unit connector 220 may comprise one or more gaskets 270 surrounding the two parts 264, 266 and the terminals 221 of the electrical control unit connector 220. The connector itself, i.e. the two parts 264, 266 may be liquid tight, e.g. by integrated metal plastic injection molding. The gasket 270 extends ensures that no liquid is able to enter the device between the electrical control unit connector 220 and the surrounding shell of the control unit. The gasket 270 is preferably made out of rubber, or another suitable flexible material.

Fig. 19 schematically illustrates an exemplary carry case 500 for the exemplary control unit 200, as described in relation to previous figures. The carry case 500 comprises a top casing 502 and a bottom casing 504 hinged along a first side 506 and connectable with a latch 510 along an opposite second side 508. An insert 512 (e.g. foam or silicone) is provided in the bottom casing 504 and being shaped to support the control unit 200.

The carry case 500 also comprises a battery space 514, e.g. as an indent in the insert 512, as illustrated, for holding a spare battery pack. In some examples, an additional or alternative battery space may be provided in the top casing 502.

The insert 512 and bottom casing 504 are adapted such that the control unit 200 is positioned allowing the battery light indicator 226 is positioned above the edge of the bottom casing 504. Thereby, the battery light indicator 226 is visibly accessible while the control unit 200 is positioned in the carry case 500. Thereby, the battery status may be checked without having to remove the control unit 200 from the carry case 500.

The insert 512 may be removable from the bottom casing 504 to facilitate cleaning of the carry case 500, as well as to facilitate replacement of the insert 512 in case needed. In the case of using foam as the insert 512. The foam may be so called skinned foam or closed cell foam such as to avoid collection of dirt in the foam. The bottom casing 504 may comprise one or more retention elements, e.g. protruding elements, such as pins or ridges to retain the insert 512 in the bottom casing.

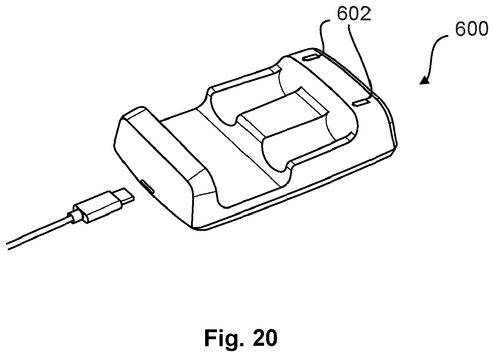

Fig. 20 schematically illustrates an exemplary battery charger 600 for a battery pack 230, as described in relation to the previous figures, e.g. Figs. 13-15. The exemplary battery charger 600 has space for charging two battery packs simultaneously. The battery charger 600 may be connected to a power supply e.g. by a USB cable, such as a USB-C cable.

The battery charger 600 comprises status indicators 602 adapted to show battery status, e.g. the state of charge, of the respective inserted battery pack. The status indicator 602 may light up in, e.g., either, orange or green signaling the state of charge. No light may indicate no charging. The status indicators 602 may be not visible when not lid. For example, light guides may be provided underneath the surface of the battery charger 600 to transmit the light from an LED to the surface.

The battery charger 600 is preferably provided without small spaces, to avoid collection of dirt, and to allow an enhanced level of cleanability.

### EXEMPLARY EMBODIMENTS

Exemplary embodiments of the present disclosure are set out in the following items:
1. A control unit for a laryngoscope,
   the control unit comprising a body portion having a first body portion and a second body portion, the first body portion being couplable to a blade unit extending from a proximal end to a distal end, the blade unit having a distal part extending to the distal end and comprising a first surface, wherein the distal part is adapted to be inserted into a patient's oral cavity with the first surface facing the patient's tongue,
   the control unit and the blade unit, when being coupled, collectively form a laryngoscope with a handle portion extending along a first axis, where a forward direction of the laryngoscope is perpendicular to the first axis in a direction towards the distal end of the blade unit and a rearward direction of the laryngoscope opposite the forward direction, the forward direction and the rearward direction extending in a laryngoscope mid-plane defined by the first axis and the distal end of the blade unit,
   the control unit being adapted to receive image data from an image sensor as the image data is being generated by the image sensor, wherein, at least when the control unit is coupled to the blade unit, the image data is indicative of a view from the distal part of the blade unit in a direction at least partly towards the distal end,
   the second body portion has an attachment part being positioned in the rearward direction relative to the first axis, and wherein the second body portion comprises a control button with a button surface being positioned in the forward direction relative to the attachment part.
2. Control unit according to item 1 wherein the button surface has a button surface normal, and wherein an angle between the button surface normal and the forward direction is less than 90 degrees, such as less than 80 degrees.
3. Control unit according to any one of items 1-2 comprising a control unit display element with a control unit display, and wherein the control unit display element is attached, such as hingedly attached, to the attachment part of the second body portion.
4. Control unit according to item 3 being adapted to cause the control unit display to display a live representation of the image data.
5. Control unit according to any one of items 1-2 comprising a connector cable adapted to connect the control unit to a monitor device, and wherein the connector cable is attached to the attachment part of the second body portion.
6. Control unit according to item 5, wherein the connector cable extends from the second body portion in a direction substantially parallel to the first axis.
7. Control unit according to any one of items 1-6, wherein the button surface comprises a tactile protrusion.
8. Control unit according to any one of items 1-7, wherein the control unit is adapted to detect a first user input corresponding to a first press of the control button, and in accordance with detection of the first user input cause recording of a first image file corresponding to the image data received when the first user input was detected.
9. Control unit according to any one of items 1-8, wherein the control unit is adapted to detect a second user input corresponding to a second press of the control button, and in accordance with detection of the second user input cause initiating a recording of a first video file corresponding to the image data received after detection of the second user input.
10. Control unit according to item 9, wherein, after detection of the second user input, the control unit is adapted to detect a third user input corresponding to the second press of the control button, and in accordance with detection of the third user input cause ending the recording of the first video file whereby the first video file corresponds to the image data received between detection of the second user input and the third user input.
11. Control unit according to any one of items 1-10, wherein the control button has a control button light indicator, and wherein the control unit is adapted to: detect coupling with the blade unit, in accordance with detection of coupling between the blade unit and the control unit provide a first visual indication with the control button light indicator, and in accordance with the detection of no coupling between the blade unit and the control unit provide a second visual indication with the control button light indicator.
12. Control unit according to any one of items 1-11 further comprising a battery being adapted to power the control unit, the battery optionally further being adapted to power the image sensor and an optional light emitter.
13. Control unit according to any one of items 1-12 comprising the image sensor and wherein the image sensor is arranged at a distal end of the first body portion.
14. Control unit according to any one of items 1-12, wherein the blade unit comprises the image sensor, and wherein the control unit comprises an electrical control unit connector at a distal end of the first body portion, wherein the electrical control unit connector is adapted to electrically connect the control unit to the image sensor of the blade unit.
15. A laryngoscope assembly comprising a blade unit extending from a proximal end to a distal end, the blade unit having a distal part extending to the distal end and comprising a first surface, wherein the distal part is adapted to be inserted into a patient's oral cavity with the first surface facing the patient's tongue,
   the laryngoscope assembly further comprising a control unit comprising a body portion having a first body portion and a second body portion, the first body portion being couplable to the blade unit,
   the control unit and the blade unit, when being coupled, collectively form a laryngoscope with a handle portion extending along a first axis, where a forward direction of the laryngoscope is perpendicular to the first axis in a direction towards the distal end of the blade unit and a rearward direction of the laryngoscope is opposite the forward direction, the forward direction and the rearward direction extending in a laryngoscope mid-plane defined by the first axis and the distal end of the blade unit,
   the control unit being adapted to receive image data from an image sensor as the image data is being generated by the image sensor, wherein, at least when the control unit is coupled to the blade unit, the image data is indicative of a view from the distal part of the blade unit in a direction at least partly towards the distal end,
   the second body portion has an attachment part being positioned in the rearward direction relative to the first axis, and wherein the second body portion comprises a control button with a button surface being positioned in the forward direction relative to the attachment part.
16. Laryngoscope assembly according to item 15 wherein the button surface has a button surface normal, and wherein an angle between the button surface normal and the forward direction is less than 90 degrees, such as less than 80 degrees.
17. Laryngoscope assembly according to any one of items 15-16, wherein the control unit comprises a control unit display element with a control unit display, and wherein the control unit display element is attached, such as hingedly attached, to the attachment part of the second body portion.
18. Control unit according to item 3 being adapted to cause the control unit display to display a live representation of the image data.
19. Laryngoscope assembly according to any one of items 15-16, wherein the control unit comprises a connector cable adapted to connect the control unit to a monitor device, and wherein the connector cable is attached to the attachment part of the second body portion.
20. Laryngoscope assembly according to item 19, wherein the connector cable extends from the second body portion in a direction substantially parallel to the first axis.
21. Laryngoscope assembly according to any one of items 15-20, wherein the button surface comprises a tactile protrusion.
22. Laryngoscope assembly according to any one of items 15-21, wherein the control unit is adapted to detect a first user input corresponding to a first press of the control button, and in accordance with detection of the first user input cause recording of a first image file corresponding to the image data received when the first user input was detected.
23. Laryngoscope assembly according to any one of items 15-22, wherein the control unit is adapted to detect a second user input corresponding to a second press of the control button, and in accordance with detection of the second user input cause initiating a recording of a first video file corresponding to the image data received after detection of the second user input.
24. Laryngoscope assembly according to item 23, wherein, after detection of the second user input, the control unit is adapted to detect a third user input corresponding to the second press of the control button, and in accordance with detection of the third user input cause ending the recording of the first video file whereby the first video file corresponds to the image data received between detection of the second user input and the third user input.
25. Laryngoscope assembly according to any one of items 15-24, wherein the control button has a control button light indicator, and wherein the control unit is adapted to: detect coupling with the blade unit, in accordance with detection of coupling between the blade unit and the control unit provide a first visual indication with the control button light indicator, and in accordance with the detection of no coupling between the blade unit and the control unit provide a second visual indication with the control button light indicator.
26. Laryngoscope assembly according to any one of items 15-25, wherein the control unit further comprises a battery being adapted to power the control unit, the battery optionally further being adapted to power the image sensor and an optional light emitter.
27. Laryngoscope assembly according to any one of items 15-26, wherein the control unit comprises the image sensor and wherein the image sensor is arranged at a distal end of the first body portion.
28. Laryngoscope assembly according to any one of items 15-26, wherein the blade unit comprises the image sensor, and wherein the control unit comprises an electrical control unit connector at a distal end of the first body portion, wherein the electrical control unit connector is adapted to electrically connect the control unit to the image sensor of the blade unit.
29. A laryngoscope assembly comprising a blade unit extending from a proximal end to a distal end, the blade unit having a distal part extending to the distal end and comprising a first surface, wherein the distal part is adapted to be inserted into a patient's oral cavity with the first surface facing the patient's tongue, the laryngoscope assembly further comprising a control unit according to any one of items 1-14.
30. A control unit for a laryngoscope,
   the control unit comprising a body portion having a first body portion and a second body portion, the first body portion being couplable to a blade unit extending from a proximal end to a distal end, the blade unit having a distal part extending to the distal end and comprising a first surface, wherein the distal part is adapted to be inserted into a patient's oral cavity with the first surface facing the patient's tongue,
   the control unit and the blade unit, when being coupled, collectively form a laryngoscope with a handle portion extending along a first axis, where a forward direction of the laryngoscope is perpendicular to the first axis in a direction towards the distal end of the blade unit and a rearward direction of the laryngoscope is opposite the forward direction, the forward direction and the rearward direction extending in a laryngoscope mid-plane defined by the first axis and the distal end of the blade unit,
   the control unit being adapted to receive image data from an image sensor as the image data is being generated by the image sensor, wherein, at least when the control unit is coupled to the blade unit, the image data is indicative of a view from the distal part of the blade unit in a direction at least partly towards the distal end,
   the control unit comprising a battery being adapted to power the control unit, the battery optionally further being adapted to power the image sensor,
   the control unit comprising a power button and a battery light indicator, and wherein the control unit is adapted to:
      - detect a first user input corresponding to a first press of the power button, and
      - in accordance with detection of the first user input provide a battery status indication with the battery light indicator, and
   wherein the control unit is further adapted to:
      - detect a second user input corresponding to a second press of the power button, and
      - in accordance with detection of the second user input turn on/off the control unit.
31. Control unit according to item 30, wherein the power button comprises the battery light indicator.
32. Control unit according to any one of items 30-31, wherein the first press of the power button corresponds to a short press of the of the power button.
33. Control unit according to any one of items 30-32, wherein the second press of the power button corresponds to a long press of the of the power button.
34. Control unit according to any one of items 30-33, wherein the battery status indication has a duration between 0.5-2.0 seconds.
35. Control unit according to any one of items 30-34, wherein the control unit is further adapted to determine a remaining battery capacity of the battery, and wherein:
   - in accordance with the remaining battery capacity being less than a low battery threshold the battery status indication comprises cyclically lighting the battery light indicator a plurality of times with a first color,
   - in accordance with the remaining battery capacity being more than the low battery threshold and less than a high battery threshold the battery status indication comprises lighting the battery light indicator once with the first color, and
   - in accordance with the remaining battery capacity being more than the high battery threshold the battery status indication comprises lighting the battery light indicator once with a second color.
36. Control unit according to any one of items 30-35 comprising a control unit display element with a control unit display, and wherein the control unit display element is attached, such as hingedly attached, to the second body portion.
37. Control unit according to item 36 being adapted to cause the control unit display to display a live representation of the image data.
38. Control unit according to any one of items 36-37 wherein the power button and/or the battery light indicator is arranged on the control unit display element.
39. A laryngoscope assembly comprising a blade unit extending from a proximal end to a distal end, the blade unit having a distal part extending to the distal end and comprising a first surface, wherein the distal part is adapted to be inserted into a patient's oral cavity with the first surface facing the patient's tongue,
   the laryngoscope assembly further comprising a control unit comprising a body portion having a first body portion and a second body portion, the first body portion being couplable to the blade unit,
   the control unit and the blade unit, when being coupled, collectively form a laryngoscope with a handle portion extending along a first axis, where a forward direction of the laryngoscope is perpendicular to the first axis in a direction towards the distal end of the blade unit and a rearward direction of the laryngoscope is opposite the forward direction, the forward direction and the rearward direction extending in a laryngoscope mid-plane defined by the first axis and the distal end of the blade unit,
   the control unit being adapted to receive image data from an image sensor as the image data is being generated by the image sensor, wherein, at least when the control unit is coupled to the blade unit, the image data is indicative of a view from the distal part of the blade unit in a direction at least partly towards the distal end,
   the control unit comprising a battery being adapted to power the control unit, the battery optionally further being adapted to power the image sensor,
   the control unit comprising a power button and a battery light indicator, and wherein the control unit is adapted to:
      - detect a first user input corresponding to a first press of the power button, and
      - in accordance with detection of the first user input provide a battery status indication with the battery light indicator, and
      wherein the control unit is further adapted to:
      - detect a second user input corresponding to a second press of the power button, and
      - in accordance with detection of the second user input turn on/off the control unit.
40. Laryngoscope assembly according to item 39, wherein the power button comprises the battery light indicator.
41. Laryngoscope assembly according to any one of items 39-40, wherein the first press of the power button corresponds to a short press of the of the power button.
42. Laryngoscope assembly according to any one of items 39-41, wherein the second press of the power button corresponds to a long press of the of the power button.
43. Laryngoscope assembly according to any one of items 39-42, wherein the battery status indication has a duration between 0.5-2.0 seconds.
44. Laryngoscope assembly according to any one of items 39-43, wherein the control unit is further adapted to determine a remaining battery capacity of the battery, and wherein:
   - in accordance with the remaining battery capacity being less than a low battery threshold the battery status indication comprises cyclically lighting the battery light indicator a plurality of times with a first color,
   - in accordance with the remaining battery capacity being more than the low battery threshold and less than a high battery threshold the battery status indication comprises lighting the battery light indicator once with the first color, and
   - in accordance with the remaining battery capacity being more than the high battery threshold the battery status indication comprises lighting the battery light indicator once with a second color.
45. Laryngoscope assembly according to any one of items 39-44, wherein the control unit comprises a control unit display element with a control unit display, and wherein the control unit display element is attached, such as hingedly attached, to the second body portion.
46. Laryngoscope assembly according to item 45, wherein the control unit is adapted to cause the control unit display to display a live representation of the image data.
47. Laryngoscope assembly according to any one of items 45-46 wherein the power button and/or the battery light indicator is arranged on the control unit display element.
48. A laryngoscope assembly comprising a blade unit extending from a proximal end to a distal end, the blade unit having a distal part extending to the distal end and comprising a first surface, wherein the distal part is adapted to be inserted into a patient's oral cavity with the first surface facing the patient's tongue, the laryngoscope assembly further comprising a control unit according to any one of items 30-38.
49. A camera unit for a laryngoscope, the camera unit comprising:
   - a camera unit housing,
   - an image sensor being adapted to generate image data indicative of a view along a viewing direction from a front surface of the image sensor, the image sensor being accommodated in a first housing compartment of the camera unit housing,
   - a light emitter adapted to emit light along the viewing direction from a front surface of the light emitter, the light emitter being accommodated in a second housing compartment of the camera unit housing separated by the first housing compartment by a separating wall.
50. Camera unit according to item 49, wherein the camera unit housing comprises a first side wall, a second side wall, a third side wall extending between the first side wall and the second side wall, and a fourth side wall extending between the first side wall and the second side wall, the camera unit housing further comprises the separating wall extending between the first side wall and the second side wall and being arranged between the third side wall and the fourth side wall, wherein the first housing compartment of the camera unit housing is defined by the first side wall, the second side wall, the third side wall and the separating wall, and wherein the second housing compartment of the camera unit housing is defined by the first side wall, the second side wall, the fourth side wall and the separating wall.
51. Camera unit according to any of items 49-50, wherein the camera unit housing comprises a front side wall, the front side wall having a first window and a second window, the separating wall adjoining the front side wall between the first window and the second window, wherein the first window provides an optical connection between the first housing compartment through the front side wall, and the second window provides an optical connection between the second housing compartment through the front side wall.
52. Camera unit according to item 51, wherein the image sensor is arranged with a distance between the front surface of the image sensor and the first window, and/or wherein the light emitter is arranged with a distance between the front surface of the light emitter and the second window.
53. Camera unit according to any of items 49-52, wherein the image sensor and the light emitter are arranged with an offset distance along the viewing direction between the front surface of the image sensor and the front surface of the light emitter.
54. Camera unit according to item 53, wherein the light emitter is arranged behind the image sensor along the viewing direction.
55. Camera unit according to any one of items 53-54, wherein the offset distance is between 0.1 mm - 1.5 mm, such as between 0.5 mm and 1 mm.
56. Camera unit according to any one of items 49-55, wherein a length of the separating wall along the viewing direction is more than 1.5 mm, such as more than 2 mm.
57. Blade unit for a laryngoscope, the blade unit extending from a proximal end to a distal end, having a distal part extending to the distal end and comprising a first surface, wherein the distal part is adapted to be inserted into a patient's oral cavity with the first surface facing the patient's tongue,
   the blade unit further comprising a camera unit according to any one of items 49-56, wherein the camera unit is arranged in the distal part with the viewing direction being at least partly towards the distal end of the blade unit.
58. Blade unit according to item 57, wherein the camera unit is arranged opposite the first surface of the distal part.
59. Blade unit according to any one of items 57-58, wherein the camera unit is arranged with a blade tip distance between the front surface of the image sensor and the distal end of the blade unit, wherein the blade tip distance is between 19-50 mm.
60. Blade unit for a laryngoscope, the blade unit extending from a proximal end to a distal end, having a distal part extending to the distal end and comprising a first surface, wherein the distal part is adapted to be inserted into a patient's oral cavity with the first surface facing the patient's tongue,
   the blade unit further comprising a camera unit comprising:
      - a camera unit housing,
      - an image sensor being adapted to generate image data indicative of a view along a viewing direction from a front surface of the image sensor, the image sensor being accommodated in a first housing compartment of the camera unit housing,
      - a light emitter adapted to emit light along the viewing direction from a front surface of the light emitter, the light emitter being accommodated in a second housing compartment of the camera unit housing separated by the first housing compartment by a separating wall,
   wherein the camera unit is arranged in the distal part with the viewing direction being at least partly towards the distal end of the blade unit.
61. Blade unit according to item 60, wherein the camera unit is arranged opposite the first surface of the distal part.
62. Blade unit according to any one of items 60-61, wherein the camera unit is arranged with a blade tip distance between the front surface of the image sensor and the distal end of the blade unit, wherein the blade tip distance is between 19-50 mm.
63. Blade unit according to any one of items 60-62, wherein the camera unit housing comprises a first side wall, a second side wall, a third side wall extending between the first side wall and the second side wall, and a fourth side wall extending between the first side wall and the second side wall, the camera unit housing further comprises the separating wall extending between the first side wall and the second side wall and being arranged between the third side wall and the fourth side wall, wherein the first housing compartment of the camera unit housing is defined by the first side wall, the second side wall, the third side wall and the separating wall, and wherein the second housing compartment of the camera unit housing is defined by the first side wall, the second side wall, the fourth side wall and the separating wall.
64. Blade unit according to any one of items 60-63, wherein the camera unit housing comprises a front side wall, the front side wall having a first window and a second window, the separating wall adjoining the front side wall between the first window and the second window, wherein the first window provides an optical connection between the first housing compartment through the front side wall, and the second window provides an optical connection between the second housing compartment through the front side wall.
65. Blade unit according to item 64, wherein the image sensor is arranged with a distance between the front surface of the image sensor and the first window, and/or wherein the light emitter is arranged with a distance between the front surface of the light emitter and the second window.
66. Blade unit according to any one of items 60-65, wherein the image sensor and the light emitter are arranged with an offset distance along the viewing direction between the front surface of the image sensor and the front surface of the light emitter.
67. Blade unit according to item 66, wherein the light emitter is arranged behind the image sensor along the viewing direction.
68. Blade unit according to any one of items 66-67, wherein the offset distance is between 0.1 mm - 1.5 mm, such as between 0.5 mm and 1 mm.
69. Blade unit according to any one of items 60-68, wherein a length of the separating wall along the viewing direction is more than 1.5 mm, such as more than 2 mm.
70. A laryngoscope assembly comprising a blade unit according to any one of items 57-69 and a control unit comprising a body portion having a first body portion and a second body portion, the first body portion being couplable to the blade unit,
   the control unit and the blade unit, when being coupled, collectively form a laryngoscope with a handle portion extending along a first axis, where a forward direction of the laryngoscope is perpendicular to the first axis in a direction towards the distal end of the blade unit and a rearward direction of the laryngoscope is opposite the forward direction, the forward direction and the rearward direction extending in a laryngoscope mid-plane defined by the first axis and the distal end of the blade unit,
   the control unit being adapted to receive image data from the image sensor as the image data is being generated by the image sensor.
71. A control unit for a laryngoscope, the control unit comprising a camera unit according to any one of items 49-56 and a body portion having a first body portion and a second body portion, wherein the first body portion is couplable to a blade unit extending from a proximal end to a distal end, the blade unit having a distal part extending to the distal end and comprising a first surface, wherein the distal part is adapted to be inserted into a patient's oral cavity with the first surface facing the patient's tongue, and
   wherein the camera unit is arranged at a distal end of the first body portion, such that when the control unit and the blade unit are coupled, the camera unit is arranged in the distal part of the blade unit with the viewing direction being in a direction at least partly towards the distal end of the blade unit.
72. A laryngoscope assembly comprising a blade unit extending from a proximal end to a distal end, the blade unit having a distal part extending to the distal end and comprising a first surface, wherein the distal part is adapted to be inserted into a patient's oral cavity with the first surface facing the patient's tongue,
   the laryngoscope assembly further comprising a control unit comprising a camera unit and a body portion having a first body portion and a second body portion, wherein the first body portion is couplable to the blade unit, and
   wherein the camera unit is arranged at a distal end of the first body portion, such that when the control unit and the blade unit are coupled, the camera unit is arranged in the distal part of the blade unit with the viewing direction being in a direction at least partly towards the distal end of the blade unit,
   the camera unit comprising:
      - a camera unit housing,
      - an image sensor being adapted to generate image data indicative of a view along a viewing direction from a front surface of the image sensor, the image sensor being accommodated in a first housing compartment of the camera unit housing,
      - a light emitter adapted to emit light along the viewing direction from a front surface of the light emitter, the light emitter being accommodated in a second housing compartment of the camera unit housing separated by the first housing compartment by a separating wall.
73. Laryngoscope assembly according to item 72, wherein the camera unit housing comprises a first side wall, a second side wall, a third side wall extending between the first side wall and the second side wall, and a fourth side wall extending between the first side wall and the second side wall, the camera unit housing further comprises the separating wall extending between the first side wall and the second side wall and being arranged between the third side wall and the fourth side wall, wherein the first housing compartment of the camera unit housing is defined by the first side wall, the second side wall, the third side wall and the separating wall, and wherein the second housing compartment of the camera unit housing is defined by the first side wall, the second side wall, the fourth side wall and the separating wall.
74. Laryngoscope assembly according to any of items 72-73, wherein the camera unit housing comprises a front side wall, the front side wall having a first window and a second window, the separating wall adjoining the front side wall between the first window and the second window, wherein the first window provides an optical connection between the first housing compartment through the front side wall, and the second window provides an optical connection between the second housing compartment through the front side wall.
75. Laryngoscope assembly according to item 74, wherein the image sensor is arranged with a distance between the front surface of the image sensor and the first window, and/or wherein the light emitter is arranged with a distance between the front surface of the light emitter and the second window.
76. Laryngoscope assembly according to any one of items 72-75, wherein the image sensor and the light emitter are arranged with an offset distance along the viewing direction between the front surface of the image sensor and the front surface of the light emitter.
77. Laryngoscope assembly according to item 76, wherein the light emitter is arranged behind the image sensor along the viewing direction.
78. Laryngoscope assembly according to any one of items 76-77, wherein the offset distance is between 0.1 mm - 1.5 mm, such as between 0.5 mm and 1 mm.
79. Laryngoscope assembly according to any one of items 72-78, wherein a length of the separating wall along the viewing direction is more than 1.5 mm, such as more than 2 mm.
80. A laryngoscope assembly comprising a blade unit extending from a proximal end to a distal end, the blade unit having a distal part extending to the distal end and comprising a first surface, wherein the distal part is adapted to be inserted into a patient's oral cavity with the first surface facing the patient's tongue,
   the laryngoscope assembly further comprising a control unit comprising a camera unit according to any one of items 49-56 and a body portion having a first body portion and a second body portion, wherein the first body portion is couplable to the blade unit, and
   wherein the camera unit is arranged at a distal end of the first body portion, such that when the control unit and the blade unit are coupled, the camera unit is arranged in the distal part of the blade unit with the viewing direction being in a direction at least partly towards the distal end of the blade unit.
81. A control unit for a laryngoscope,
   the control unit comprising a body portion having a first body portion and a second body portion, the first body portion being couplable to a blade unit extending from a proximal end to a distal end, the blade unit having a distal part extending to the distal end and comprising a first surface, wherein the distal part is adapted to be inserted into a patient's oral cavity with the first surface facing the patient's tongue,
   the control unit and the blade unit, when being coupled, collectively form a laryngoscope with a handle portion extending along a first axis, where a forward direction of the laryngoscope is perpendicular to the first axis in a direction towards the distal end of the blade unit and a rearward direction of the laryngoscope is opposite the forward direction, the forward direction and the rearward direction extending in a laryngoscope mid-plane defined by the first axis and the distal end of the blade unit,
   the control unit being adapted to receive image data from an image sensor as the image data is being generated by the image sensor, wherein, at least when the control unit is coupled to the blade unit, the image data is indicative of a view from the distal part of the blade unit in a direction at least partly towards the distal end,
   the control unit comprising a battery receiver adapted to receive a replaceable battery pack adapted to power the control unit, the battery receiver comprising a battery connector with a plurality of battery connector terminals adapted to cooperate with corresponding connection terminals of the replaceable battery pack,
   the battery receiver comprising an outer connector seal encircling the battery connector and being adapted to, when the replaceable battery pack is received in the battery receiver, contact a corresponding surface of the replaceable battery pack encircling the connection terminals of the replaceable battery pack.
82. Control unit according to item 81, wherein the replaceable battery pack is devoid of an outer sealing element adapted to contact a surface of the control unit.
83. Control unit according to any one of items 81-82, wherein the control unit comprises an internal sealing encircling the battery connector and preventing liquid from entering from the battery receiver and into an internal space of the control unit when the replaceable battery pack is not received in the battery receiver.
84. Control unit according to item 83 comprising a battery connector circuitry, the battery connector being attached to a first side of the battery connector circuity, the internal sealing being arranged between the battery connector circuitry and a shell element of the control unit forming part of the battery receiver.
85. Control unit according to any one of items 81-84, wherein the replaceable battery pack comprises a rechargeable battery.
86. Control unit according to any one of items 81-85, wherein the battery receiver forms part of the first body portion, and wherein the first body portion is adapted to be received and enclosed by a handle portion of the blade unit.
87. A laryngoscope assembly comprising a blade unit extending from a proximal end to a distal end, the blade unit having a distal part extending to the distal end and comprising a first surface, wherein the distal part is adapted to be inserted into a patient's oral cavity with the first surface facing the patient's tongue,
   the laryngoscope assembly further comprising a control unit comprising a body portion having a first body portion and a second body portion, the first body portion being couplable to the blade unit,
   the control unit and the blade unit, when being coupled, collectively form a laryngoscope with a handle portion extending along a first axis, where a forward direction of the laryngoscope is perpendicular to the first axis in a direction towards the distal end of the blade unit and a rearward direction of the laryngoscope is opposite the forward direction, the forward direction and the rearward direction extending in a laryngoscope mid-plane defined by the first axis and the distal end of the blade unit,
   the control unit being adapted to receive image data from an image sensor as the image data is being generated by the image sensor, wherein, at least when the control unit is coupled to the blade unit, the image data is indicative of a view from the distal part of the blade unit in a direction at least partly towards the distal end,
   the control unit comprising a battery receiver adapted to receive a replaceable battery pack adapted to power the control unit, the battery receiver comprising a battery connector with a plurality of battery connector terminals adapted to cooperate with corresponding connection terminals of the replaceable battery pack,
   the battery receiver comprising an outer connector seal encircling the battery connector and being adapted to, when the replaceable battery pack is received in the battery receiver, contact a corresponding surface of the replaceable battery pack encircling the connection terminals of the replaceable battery pack.
88. Laryngoscope assembly according to item 87 the replaceable battery pack is devoid of an outer sealing element adapted to contact a surface of the control unit.
89. Laryngoscope assembly according to any one of items 87-88, wherein the control unit comprises an internal sealing encircling the battery connector and preventing liquid from entering from the battery receiver and into an internal space of the control unit when the replaceable battery pack is not received in the battery receiver.
90. Laryngoscope assembly according to item 89, wherein the control unit comprises a battery connector circuitry, the battery connector being attached to a first side of the battery connector circuity, the internal sealing being arranged between the battery connector circuitry and a shell element of the control unit forming part of the battery receiver.
91. Laryngoscope assembly according to any one of items 87-90, wherein the replaceable battery pack comprises a rechargeable battery.
92. Laryngoscope assembly according to any one of items 87-91, wherein the battery receiver forms part of the first body portion, and wherein the first body portion is adapted to be received and enclosed by a handle portion of the blade unit.
93. A laryngoscope assembly comprising a blade unit extending from a proximal end to a distal end, the blade unit having a distal part extending to the distal end and comprising a first surface, wherein the distal part is adapted to be inserted into a patient's oral cavity with the first surface facing the patient's tongue, the laryngoscope assembly further comprising a control unit according to any one of items 81-86.
94. A blade unit for a laryngoscope, the blade unit extending from a proximal end to a distal end, and having a distal part extending to the distal end and comprising a first surface, wherein the distal part is adapted to be inserted into a patient's oral cavity with the first surface facing the patient's tongue,
   the blade unit at the proximal end comprising a blade coupling portion adapted to couple the blade unit to a control unit to form the laryngoscope, the blade coupling portion comprising a channel extending along a channel axis to receive a first body portion of the control unit, wherein the channel is formed of a first channel part and a second channel part, the second channel part being between the proximal end of the blade unit and the first channel part, the first channel part and the second channel part being connected along a connected portion between a first angular position and a second angular position about the channel axis, and being separated along a slit portion extending from the second angular position to the first angular position about the channel axis,
   wherein the second channel part comprises at least one retention element between a first retention element angular position and a second retention element angular position about the channel axis, the first retention element angular position and the second retention element angular position being within the slit portion, and wherein an angular distance between the first retention element angular position and the first angular position is at least 45 degrees and an angular distance between the second retention element angular position and the second angular position is at least 45 degrees.
95. Blade unit according to item 94, wherein the angular distance between the first retention element angular position and the first angular position and the angular distance between the second retention element angular position and the second angular position is substantially the same.
96. Blade unit according to any one of items 94-95, wherein the slit portion is longer than the connected portion.
97. Blade unit according to any one of items 94-96, wherein the angular distance between the first retention element angular position and the first angular position is at least 70 degrees and/or the angular distance between the second retention element angular position and the second angular position is at least 70 degrees.
98. Blade unit according to any one of items 94-97 comprising an image sensor being arranged in the distal part with a viewing direction being in a direction at least partly towards the distal end of the blade unit.
99. Blade unit according to item 98 comprising an electrical blade connector comprising one or more terminals electrically connected to the image sensor, the electrical blade connector being adapted to electrically connect the image sensor with the control unit couplable to the blade unit, and wherein the electrical blade connector is arranged in the channel, preferably at a distal end of the channel opposite the proximal end of the blade unit.
100. Blade unit according to any one of items 94-99, wherein the first channel part comprises one or more alignment features adapted to engage with one or more cooperating alignment features of the first body portion of the control unit, preferably wherein the one or more alignment features are one or more protrusions.
101. A control unit for a laryngoscope,
   the control unit comprising a body portion having a first body portion and a second body portion, the first body portion being couplable to a blade unit according to any one of items 94-100,
   the first body portion extends along a first body portion axis, and wherein when the first body portion is received in the channel of the blade unit the first body portion axis is substantially parallel to the channel axis, and
   the first body portion comprising one or more cooperating retention elements adapted to engage with the at least one retention element of the second channel part.
102. Control unit according to item 101, wherein the first body portion has a first width at a first position between a proximal end of the first body portion adjoining the second body portion and an intermediate position along the first body portion axis, and wherein the first body portion has a second width greater than the first width at a second position between the intermediate position and a distal position along the first body portion axis.
103. Control unit according to any of items 101-102, wherein the first body portion comprises one or more cooperating alignment features adapted to engage with the one or more alignment features of the first channel part, preferably wherein the one or more cooperating alignment features are slits extending along the first body portion axis.
104. Control unit according to item 103 as dependent on item 102, wherein the one or more cooperating alignment features are provided between the intermediate position and the distal position along the first body portion axis.
105. A laryngoscope assembly comprising a blade unit according to any one of items 94-100 and a control unit comprising a body portion having a first body portion and a second body portion, the first body portion being couplable to the blade unit,
   the first body portion extends along a first body portion axis, and wherein when the first body portion is received in the channel of the blade unit the first body portion axis is substantially parallel to the channel axis, and
   the first body portion comprising one or more cooperating retention elements adapted to engage with the at least one retention element of the second channel part.
106. Laryngoscope assembly according to item 105, wherein the first body portion has a first width at a first position between a proximal end of the first body portion adjoining the second body portion and an intermediate position along the first body portion axis, and wherein the first body portion has a second width greater than the first width at a second position between the intermediate position and a distal position along the first body portion axis.
107. Laryngoscope assembly according to any one of items 105-106, wherein the first body portion comprises one or more cooperating alignment features adapted to engage with the one or more alignment features of the first channel part, preferably wherein the one or more cooperating alignment features are slits extending along the first body portion axis.
108. Laryngoscope assembly according to item 107 as dependent on item 106, wherein the one or more cooperating alignment features are provided between the intermediate position and the distal position along the first body portion axis.
109. A laryngoscope assembly comprising a blade unit according to any one of items 94-100 and a control unit according to any one of items 101-104.
110. A laryngoscope system comprising:
   - a blade unit extending from a proximal end to a distal end, and having a distal part extending to the distal end and comprising a first surface, wherein the distal part is adapted to be inserted into a patient's oral cavity with the first surface facing the patient's tongue,
   - an image sensor adapted to generate image data indicative of a view in a viewing direction from the distal part of the blade unit in a direction at least partly towards the distal end, and
   - a display, and
   wherein the laryngoscope system:
   - displays a live representation of the image data with the display,
   - detects a trigger-event, and
   - in response to detection of the trigger event:
      - starts a timer function, and
      - displays, concurrently with the live representation, a value of the timer function with the display.
111. Laryngoscope system according to item 110 comprising a control unit comprising a body portion having a first body portion couplable to the blade unit, the control unit further comprising the display.
112. Laryngoscope system according to item 111, wherein the control unit comprises a control unit display element with the display.
113. Laryngoscope system according to any one of items 110-112, wherein the laryngoscope system detects coupling between the control unit and the blade unit, and wherein the trigger event corresponds to coupling of the blade unit and the control unit.
114. Laryngoscope system according to any one of items 110-113, wherein the blade unit comprises the image sensor being arranged in the distal part of the blade unit.
115. Laryngoscope system according to any one of items 110-114, wherein the trigger event corresponds to a user input.
116. Laryngoscope system according to any one of items 110-114, wherein the laryngoscope system detects, by image analysis of the image data, insertion of the distal part of the blade unit into a patient's oral cavity, and wherein the trigger event corresponds to insertion of the distal part of the blade unit into a patient's oral cavity.
117. Laryngoscope system according to any one of items 110-116, wherein the timer function is a countdown from a pre-defined amount of time, e.g. 30 seconds.
118. Laryngoscope system according to any one of items 110-117, wherein, after start of the timer function, in accordance with the value of the timer function being outside a low-threshold time range, the laryngoscope system displays the value of the timer function having a first visual characteristic, and in accordance with the value of the timer function being within the low-threshold time range, the laryngoscope system displays the value of the timer function having a second visual characteristic different from the first visual characteristic.

The invention has been described with reference to preferred embodiments. However, the scope of the invention is not limited to the illustrated embodiments, and alterations and modifications can be carried out without deviating from the scope of the invention.

Throughout the description, the use of the terms "first", "second", "third", "fourth", "primary", "secondary", "tertiary" etc. does not imply any particular order of importance but are included to identify individual elements. Furthermore, the labelling of a first element does not imply the presence of a second element and vice versa.

### LIST OF REFERENCES

- 2: laryngoscope system
- 4, 4': laryngoscope assembly
- 6, 6': laryngoscope

- 100: blade unit
- 102: proximal end
- 104: distal end
- 106: distal part
- 108: first surface
- 110: handle portion
- 112: electrical blade connector
- 113: terminals
- 114: blade coupling portion
- 116: channel
- 116A: first channel part
- 116B: second channel part
- 117: distal end of channel
- 118: connected portion
- 120: slit portion
- 122A: first angular position
- 122B: second angular position
- 124: retention element
- 126A: first retention element angular position
- 126B: second retention element angular position
- 128: alignment features
- 130: pinch point indicator
- 132: curved end
- dA1: angular distance between first retention element angular position and first angular position
- dA2: angular distance between second retention element angular position and second angular position

- 200, 200': control unit
- 202: body portion
- 202A: first body portion
- 202B: second body portion
- 203: distal end of first body portion
- 204: attachment part
- 205: ramp
- 206: control button
- 207: tactile protrusion
- 208: button surface
- 208n: button surface normal
- 209: control button light indicator
- 210: control unit display element
- 212: control unit display
- 213: live representation
- 214: hinge
- 215: cable interface
- 216: connector cable
- 217: extension direction of connector cable
- 218: control unit processing unit
- 219: memory
- 220: electrical control unit connector
- 221: terminals
- 222: battery
- 224: power button
- 226: battery light indicator
- 228: battery receiver
- 230: battery pack
- 232: battery connector
- 234: battery connector terminals
- 236: connection terminals
- 238: outer connector seal
- 240: surface of battery pack
- 242: internal sealing
- 244: battery connector circuitry
- 246: shell element
- 248: cooperating retention element
- 250: cooperating alignment feature
- w1: width at first position
- 252: proximal end of first body portion
- 254: intermediate position
- w2: width at second position
- 256: distal position
- 258: extension portion
- 260: light sensor
- 262: alignment feature
- 264: first part of electrical blade connector
- 266: second part of electrical blade connector
- 268: retention element
- 270: gasket

- 300: monitor device
- 302: monitor display
- 304: monitor processing unit
- 305: monitor memory
- 306: monitor connector

- 400: camera unit
- 401: electrical camera connector
- 402: image sensor
- 404: light emitter
- 410: camera unit housing
- 411: first side wall
- 412: second side wall
- 413: third side wall
- 414: fourth side wall
- 415: separating wall
- 415L: length of separating wall
- 416A: first housing compartment
- 416B: second housing compartment
- 418: front surface of image sensor
- 420: front surface of light emitter
- 422: front side wall
- 424A: first window
- 424B: second window
- d1: distance between front surface of image sensor and first window
- d2: distance between front surface of light emitter and second window
- df: offset distance
- db: blade tip distance

- 500: carry case
- 502: top casing
- 504: bottom casing
- 506: first side
- 508: second side
- 510: latch
- 512: insert

- 600: battery charger
- 602: status indicator

- Ax1: first axis
- AxH: hinge axis
- AxC: channel axis
- AxB: body portion axis
- Fw: forward direction
- Rw: rearward direction
- V: viewing direction

## Claims

1. Blade unit for a laryngoscope, the blade unit extending from a proximal end to a distal end, having a distal part extending to the distal end and comprising a first surface, wherein the distal part is adapted to be inserted into a patient's oral cavity with the first surface facing the patient's tongue, the blade unit further comprising a camera unit comprising:
- a camera unit housing,
- an image sensor being adapted to generate image data indicative of a view along a viewing direction from a front surface of the image sensor, the image sensor being accommodated in a first housing compartment of the camera unit housing,
- a light emitter adapted to emit light along the viewing direction from a front surface of the light emitter, the light emitter being accommodated in a second housing compartment of the camera unit housing separated by the first housing compartment by a separating wall,
wherein the camera unit is arranged in the distal part with the viewing direction being at least partly towards the distal end of the blade unit.

2. Blade unit according to claim 1, wherein the camera unit is arranged opposite the first surface of the distal part.

3. Blade unit according to any one of claims 1-2, wherein the camera unit is arranged with a blade tip distance between the front surface of the image sensor and the distal end of the blade unit, wherein the blade tip distance is between 19-50 mm.

4. Blade unit according to any one of claims 1-3, wherein the camera unit housing comprises a first side wall, a second side wall, a third side wall extending between the first side wall and the second side wall, and a fourth side wall extending between the first side wall and the second side wall, the camera unit housing further comprises the separating wall extending between the first side wall and the second side wall and being arranged between the third side wall and the fourth side wall, wherein the first housing compartment of the camera unit housing is defined by the first side wall, the second side wall, the third side wall and the separating wall, and wherein the second housing compartment of the camera unit housing is defined by the first side wall, the second side wall, the fourth side wall and the separating wall.

5. Blade unit according to any one of claims 1-4 wherein the camera unit housing comprises a front side wall, the front side wall having a first window and a second window, the separating wall adjoining the front side wall between the first window and the second window, wherein the first window provides an optical connection between the first housing compartment through the front side wall, and the second window provides an optical connection between the second housing compartment through the front side wall.

6. Blade unit according to claim 5, wherein the image sensor is arranged with a distance between the front surface of the image sensor and the first window, and/or wherein the light emitter is arranged with a distance between the front surface of the light emitter and the second window.

7. Blade unit according to any one of claims 1-6, wherein the image sensor and the light emitter are arranged with an offset distance along the viewing direction between the front surface of the image sensor and the front surface of the light emitter.

8. Blade unit according to claim 7, wherein the light emitter is arranged behind the image sensor along the viewing direction.

9. Blade unit according to any one of claims 7-8, wherein the offset distance is between 0.1 mm - 1.5 mm, such as between 0.5 mm and 1 mm.

10. Blade unit according to any one of claims 1-9, wherein a length of the separating wall along the viewing direction is more than 1.5 mm, such as more than 2 mm.

11. A laryngoscope assembly comprising a blade unit according to any one of claims 1-10 and a control unit comprising a body portion having a first body portion and a second body portion, the first body portion being couplable to the blade unit,
the control unit and the blade unit, when being coupled, collectively form a laryngoscope with a handle portion extending along a first axis, where a forward direction of the laryngoscope is perpendicular to the first axis in a direction towards the distal end of the blade unit and a rearward direction of the laryngoscope is opposite the forward direction, the forward direction and the rearward direction extending in a laryngoscope mid-plane defined by the first axis and the distal end of the blade unit,
the control unit being adapted to receive image data from the image sensor as the image data is being generated by the image sensor.

12. A laryngoscope assembly comprising a blade unit extending from a proximal end to a distal end, the blade unit having a distal part extending to the distal end and comprising a first surface, wherein the distal part is adapted to be inserted into a patient's oral cavity with the first surface facing the patient's tongue,
the laryngoscope assembly further comprising a control unit comprising a camera unit and a body portion having a first body portion and a second body portion, wherein the first body portion is couplable to the blade unit, and
wherein the camera unit is arranged at a distal end of the first body portion, such that when the control unit and the blade unit are coupled, the camera unit is arranged in the distal part of the blade unit with the viewing direction being in a direction at least partly towards the distal end of the blade unit,
the camera unit comprising:
- a camera unit housing,
- an image sensor being adapted to generate image data indicative of a view along a viewing direction from a front surface of the image sensor, the image sensor being accommodated in a first housing compartment of the camera unit housing,
- a light emitter adapted to emit light along the viewing direction from a front surface of the light emitter, the light emitter being accommodated in a second housing compartment of the camera unit housing separated by the first housing compartment by a separating wall.

13. Laryngoscope assembly according to claim 12, wherein the camera unit housing comprises a first side wall, a second side wall, a third side wall extending between the first side wall and the second side wall, and a fourth side wall extending between the first side wall and the second side wall, the camera unit housing further comprises the separating wall extending between the first side wall and the second side wall and being arranged between the third side wall and the fourth side wall, wherein the first housing compartment of the camera unit housing is defined by the first side wall, the second side wall, the third side wall and the separating wall, and wherein the second housing compartment of the camera unit housing is defined by the first side wall, the second side wall, the fourth side wall and the separating wall.

14. Laryngoscope assembly according to any of claims 12-13, wherein the camera unit housing comprises a front side wall, the front side wall having a first window and a second window, the separating wall adjoining the front side wall between the first window and the second window, wherein the first window provides an optical connection between the first housing compartment through the front side wall, and the second window provides an optical connection between the second housing compartment through the front side wall.

15. Laryngoscope assembly according to claim 14, wherein the image sensor is arranged with a distance between the front surface of the image sensor and the first window, and/or wherein the light emitter is arranged with a distance between the front surface of the light emitter and the second window.

16. Laryngoscope assembly according to any one of claims 12-15, wherein the image sensor and the light emitter are arranged with an offset distance along the viewing direction between the front surface of the image sensor and the front surface of the light emitter.

17. Laryngoscope assembly according to claim 16, wherein the light emitter is arranged behind the image sensor along the viewing direction.

18. Laryngoscope assembly according to any one of claims 16-17, wherein the offset distance is between 0.1 mm - 1.5 mm, such as between 0.5 mm and 1 mm.

19. Laryngoscope assembly according to any one of claims 12-18, wherein a length of the separating wall along the viewing direction is more than 1.5 mm, such as more than 2 mm.
